# EUROPEAN PATENT SPECIFICATION

(11) **EP 1 948 176 B1**
(45) Date of publication and mention of the grant of the patent: **05.01.2011**
(21) Application number: 06828882.8
(22) Date of filing: 30.10.2006
(51) Int. Cl.: A61K 31/495, A61P 11/00

(54) **DIARYL UREAS FOR TREATING PULMONARY HYPERTENSION**
DIARYLHARNSTOFFE ZUR BEHANDLUNG VON PULMONALER HYPERTONIE
DIARYLE-UREES PERMETTANT DE TRAITER L'HYPERTENSION PULMONAIRE

(30) Priority: 10.11.2005 EP 05024508; 15.12.2005 EP 05027449; 13.04.2006 EP 06007775
(43) Date of publication of application: 30.07.2008
(73) Proprietor: Bayer Schering Pharma Aktiengesellschaft, 13353 Berlin (DE)
(72) Inventor: SANDNER, Peter, 42113 Wuppertal (DE); TINEL, Hanna, 42111 Wuppertal (DE); HÜTTER, Joachim, 42349 Wuppertal (DE); RIEDL, Bernd, 42329 Wuppertal (DE); KLEIN, Martina, 40489 Düsseldorf (DE); SCHERMULY, Ralph, 35794 Mengerskirchen (DE); GRIMMINGER, Friedrich, 35510 Butzbach (DE)
(86) International application number: PCT/EP2006/010405
(87) International publication number: WO 2007/054215

(56) References cited:
- WO-A-03/068228
- WO-A2-2004/113274

## Description

The present invention relates to pharmaceutical compositions for treating, preventing or managing pulmonary hypertension comprising at least a diaryl urea compound optionally combined with at least one additional therapeutic agent. Useful combinations include e.g. BAY 43-9006 as a diaryl urea compound.

BAY 43-9006 refers to 4{4-[3-(4-chloro-3-trifluoromethylphenyl)-ureido]-phenoxy}-pyridine-2-carboxylic acid methyl amide and is species of diaryl urea compounds which are potent anti-cancer and anti-angiogenic agents that possess various activities, including inhibitory activity on the VEGFR, PDGFR, raf, p38, and/or flt-3 kinase signaling molecules. See, e.g., WO 2004/113274 and WO 2005/000284.

Pulmonary hypertension refers to a disease characterized by sustained elevations of pulmonary artery pressure (L.J. Rubin, The New England Journal of Medicine,1997, 336(2), 111). Current treatment of pulmonary hypertension depends on the stage and the mechanism of the disease. Typical treatments for pulmonary hypertension include anticoagulation, oxygen supplementation, conventional vasodilator therapy, transplantation and surgical care. Therapeutic agents presently used for the treatment of pulmonary hypertension include e.g. calcium channel blockers and pulmonary vasodilators

The present invention provides pharmaceutical compositions for treating, preventing or managing pulmonary hypertension comprising at least one compound of formula I and optionally at least one further therapeutic agent.

The present invention can be used e.g. by administering a diaryl urea compound of formula I and optionally a further therapeutic agent, pharmaceutically-acceptable salts thereof, and derivatives thereof, etc.

The compounds with the structure of formula (I), pharmaceutically acceptable salts, polymorphs, solvates, hydrates metabolites and prodrugs thereof, including diastereoisomeric forms (both isolated stereoisomers and mixtures of stereoisomers) are collectively referred to herein as the "compounds of formula I".

Formula (I) is as follows: 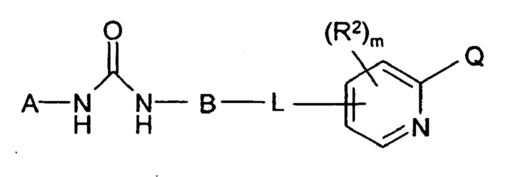 wherein
- Q: is -C(O)Rₓ
- Rₓ: is hydroxy, C₁₋₄ alkyl, C₁₋₄ alkoxy or NRₐR_{b},
- Rₐ and R_{b}: are independently :
- a): hydrogen;
- b): C₁₋₄ alkyl, optionally substituted by
-hydroxy,
-C₁₋₄ alkoxy,
- a heteroaryl group selected from pyrrole, furan, thiophene,imidazole, pyrazole, thiazole, oxazole, isoxazole, isothiazole, triazole, tetrazole, thiadiazole, oxadiazole, pyridine, pyrimidine, pyridazine, pyrazine, triazine, benzoxazole, isoquioline, quinolines and imidazopyrimidine
- a heterocyclic group selected from tetrahydropyran, tetrahydrofuran, 1,3- dioxolane, 1,4-dioxane, morpholine, thiomorpholine, piperazine, piperidine, piperidinone, tetrahydropyrimidone, pentamethylene sulfide, tetramethylene sulfide, dihydropyrane, dihydrofuran, and dihydrothiophene,
- amino, -NH₂, optionally substituted by one or two C₁₋₄alkyl groups, or
- phenyl,
- c): phenyl optionally substituted with
- halogen, or
- amino, -NH₂, optionally substituted by one or two C₁₋₄ alkyl, or
- d): - a heteroaryl group selected from pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, triazine, benzoxazole, isoquioline, quinoline and imidazopyrimidine;
- A: is optionally substituted phenyl, pyridinyl, naphthyl, benzoxazole, isoquioline, quinoline or imidazopyrimidine;
- B: is optionally substituted phenyl or naphthyl:
- L: is a bridging group which is -S- or -O-;
- m: is 0,1,2 or 3, and
each R² is independently C₁₋₅ alkyl, C₁₋₅ haloalkyl, C₁₋₃ alkoxy, N-oxo or N-hydroxy.

Structures of optionally substituted phenyl moieties for A of formula (I) which are of particular interest include structures of formula 1xx:

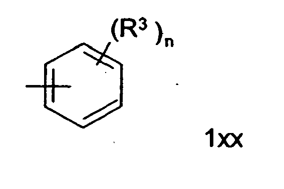

Structures of optionally substituted pyridinyl moieties for A of formula (I) which are of particular interest include structures of formula 1x:

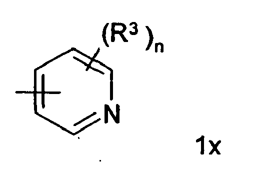

Structures of optionally substituted naphthyl moieties for A of formula (I) which are of particular interest include structures of formula 1y:

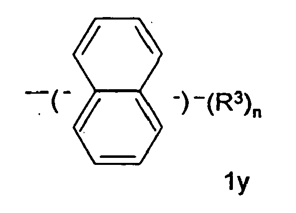

The structure 1y represents that the substituents R³ can appear on any carbon atom in either ring which has a valence that is otherwise complete with a hydrogen atom as a substituent. The bond to the urea group can also be through any carbon atom on either ring which has a valence that is otherwise complete with a hydrogen atom as a substituent.

B is optionally substituted phenyl or naphthyl. Structures of optionally substituted phenyl or naphthyl moieties for B of formula (I) which are of particular interest include structures 2a and 2b:

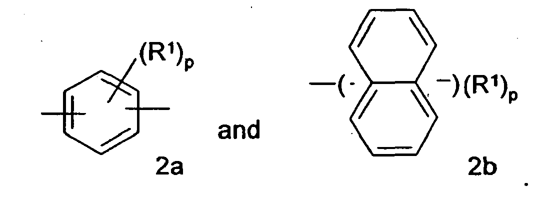

The structures 2a and 2b represent that the substituents R¹ can appear on any carbon atom in the structure which has a valence that is otherwise complete with a hydrogen atom as a substituent and the bond to the urea group can be through any carbon atom in the structure which has a valence that is otherwise complete with a hydrogen atom as a substituent.

In a class of embodiments of this invention, B is substituted by at least one halogen substituent. In another class of embodiments, Rₓ is NRₐR_{b}, and Rₐ and R_{b} are independently hydrogen or C₁₋₄ alkyl optionally substituted by hydroxy and L is a bridging group which is -S- or -O-.

The variable p is 0, 1, 2, 3, or 4, typically 0 or 1. The variable n is 0, 1, 2, 3, 4, 5 or 6, typically 0,1,2,3 or 4. The variable m is 0,1,2 or 3, typically 0.

Each R¹ is independently: halogen, C₁₋₅haloalkyl NO₂, C(O)NR⁴R⁵, C₁₋₆ alkyl, C₁₋₆ dialkylamine, C₁₋₃ alkylamine, CN, amino, hydroxy or C₁₋₃ alkoxy. Where present, R¹ is more commonly halogen and of the halogens, typically chlorine or fluorine, and more commonly fluorine.

Each R² is independently: C₁₋₅ alkyl, C₁₋₅ haloalkyl, C₁₋₃ alkoxy, N-oxo or N-hydroxy. Where present, R² is typically methyl or trifluoromethyl.

Each R³ is independently selected from halogen, R⁴, OR⁴, S(O)R⁴, C(O)R⁴, C(O)NR⁴R⁵,oxo, cyano or nitro (NO₂).

R⁴ and R⁵ are independently selected from hydrogen, C₁₋₆ alkyl, and up to per-halogenated C₁₋₆ alkyl.

Other examples of A include: 3-tert butyl phenyl, 5-tert butyl-2-methoxyphenyl,

5-(trifluoromethyl)-2 phenyl, 3-(trifluoromethyl -4 chlorophenyl, 3-(trifluoromethyl)4-bromophenyl and 5-(trifluoromethyl)-4-chloro-2 methoxyphenyl.

Other examples of B include:

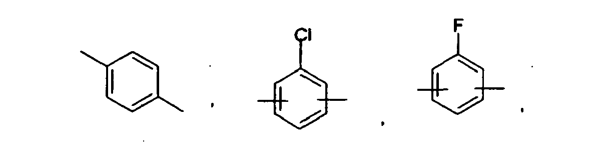

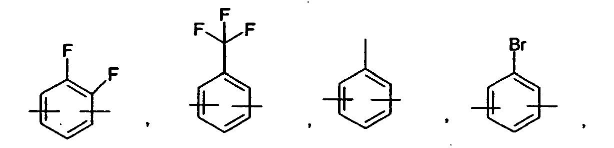

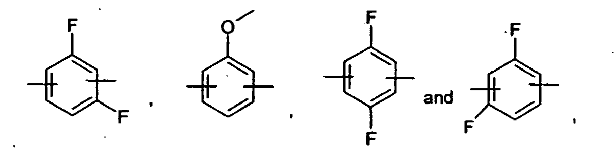

Preferably the urea group -NH-C(O)-NH- and the bridging group, L, are not bound to contiguous ring carbons of B, but rather have 1 or 2 ring carbons separating them.

Examples of R¹ groups include fluorine, chorine, bromine, methyl, NO₂, C(O)NH₂, methoxy, SCH₃, trifluoromethyl, and methanesulfonyl.

Examples of R² groups include methyl, ethyl, propyl, oxygen, and cyano.

Examples of R³ groups include trifluoromethyl, methyl, ethyl, propyl, butyl, isopropyl, tert-butyl, chlorine, fluorine, bromine, cyano, methoxy, acetyl, trifluoromethanesulfonyl, trifluoromethoxy, and trifluoromethylthio.

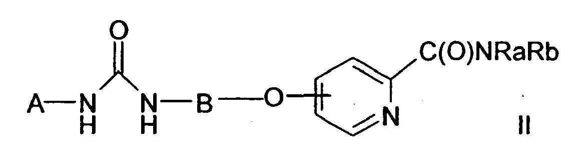

A class of compounds of interest are of formula II below
wherein Ra and Rb are independently hydrogen and C₁-C₄ alkyl,
B of formula II is 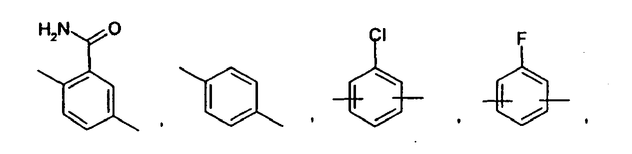 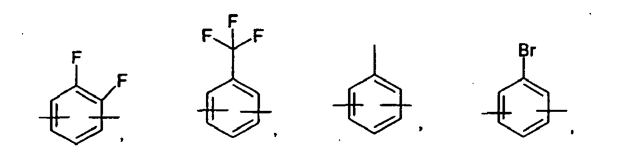 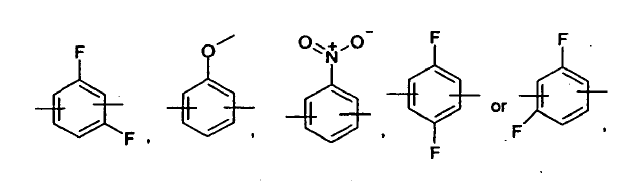 wherein the urea group, -NH-C(O)-NH-, and the oxygen bridging group are not bound to contiguous ring carbons of B, but rather have 1 or 2 ring carbons separating them,
and A of formula (II) is 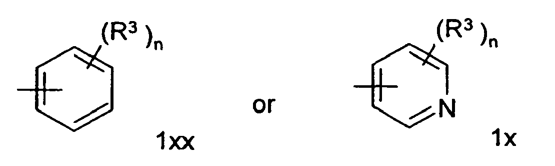 wherein the variable n is 0, 1, 2, 3 or 4.

R³ is trifluoromethyl, methyl, ethyl, propyl, butyl, isopropyl, tert-butyl, chlorine, fluorine, bromine, cyano, methoxy, acetyl, trifluoromethanesulfonyl, trifluoromethoxy, or trifluoromethylthio.

In a subclass of such compounds, each R³ substituent on A of formula II is selected from chlorine, trifluoromethyl, tert-butyl or methoxy.

In another subclass of such compounds, A of formula II is 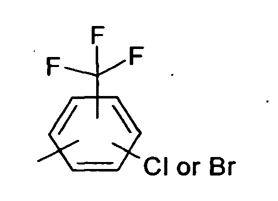 and B of formula II is phenylene, fluoro substituted phenylene or difluoro substituted phenylene.

Another class of compounds of interest includes compounds having the structure of formulae X below wherein phenyl ring "B" optionally has one halogen substituent.

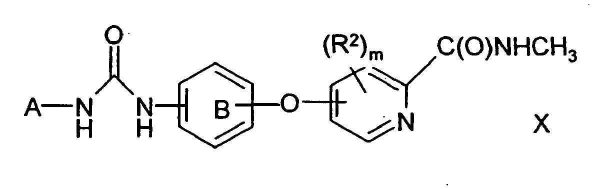

For the compounds of formula X, R², m and A are as defined above for formula I. The variable "m" is preferably zero, leaving C(O)NHCH₃ as the only substituent on the pyridinyl moiety. Preferred values for A are substituted phenyl which have at least one substituent, R³. R³ is preferably halogen, preferably Cl or F, trifluoromethyl and/or methoxy.

A subclass of compounds of interest includes compounds having the structure of formulas Z1 and Z2 below :

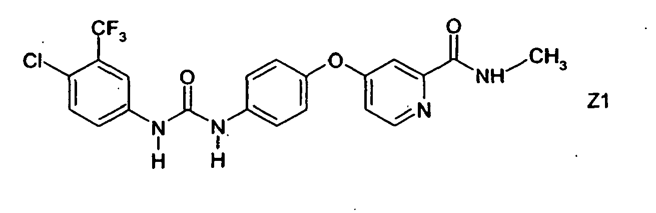

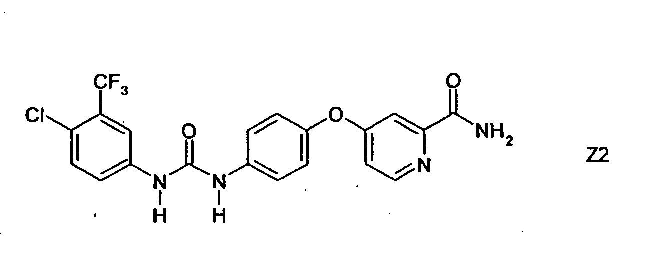

Preferably used as compound of formula I according to the invention is 4{4-[3-(4-chloro-3-trifluoromethylphenyl)-ureido]-phenoxy}-pyridine-2-carboxylic acid methyl amide (BAY 43-9006) or the *p*-toluenesulfonic acid salt of 4{4-[3-(4-chloro-3-trifluoromethylphenyl)-ureido]-phenoxy}-pyridine-2-carboxylic acid methyl amide (tosylate salt of compound (I)). More preferably the *p*-toluenesulfonic acid salt of 4{4-[3-(4-chloro-3-trifluoromethylphenyl)-ureido]-phenoxy}-pyridine-2-carboxylic acid methyl amide exists for at least 80% in the stable polymorph I. Most preferably the *p*-toluenesulfonic acid salt of 4{4-[3-(4-chloro-3-trifluoromethylphenyl)-ureido]-phenoxy}-pyridine-2-carboxylic acid methyl amide exists for at least 80% in the stable polymorph I and in a micronized form.

Micronization can be achieved by standard milling methods, preferably by air chat milling, known to a skilled person. The micronized form can have a mean particle size of from 0.5 to 10 µm, preferably from 1 to 6 µm, more preferably from 1 to 3 µm. The indicated particle size is the mean of the particle size distribution measured by laser diffraction known to a skilled person (measuring device: HELOS, Sympatec).

The process for preparing the *p*-toluenesulfonic acid salt of 4{4-[3-(4-chloro-3-trifluoromethylphenyl)-ureido]-phenoxy}-pyridine-2-carboxylic acid methyl amide and its stable polymorph I are described in the patent applications EP 04023131.8 and EP 04023130.0.

When any moiety is "substituted", it can have up to the highest number of indicated substituents and each substituent can be located at any available position on the moiety and can be attached through any available atom on the substituent. "Any available position" means any position on the moiety that is chemically accessible through means known in the art or taught herein and that does not create an unstable molecule, e.g., incapable of administration to a human. When there are two or more substituents on any moiety, each substituent is defined independently of any other substituent and can, accordingly, be the same or different.

The term "optionally substituted" means that the moiety so modified may be either unsubstituted, or substituted with the identified substituent(s).

It is understood that the term "hydroxy" as a pyridine substituent includes 2-, 3-, and 4-hydroxypyridine, and also includes those structures referred to in the art as 1-oxo-pyridine, 1-hydroxy-pyridine or pyridine N-oxide:
Where the plural form of the word compounds, salts, and the like, is used herein, this is taken to mean also a single compound, salt, or the like.

The term C₁₋₆ alkyl, unless indicated otherwise, means straight, branched chain or cyclic alkyl groups having from one to six carbon atoms, which may be cyclic, linear or branched with single or multiple branching. Such groups include for example methyl, ethyl, *n*-propyl, isopropyl, *n-*butyl, isobutyl, *sec*-butyl, *tert*-butyl, cyclopropyl, cyclobutyl and the like.

The term C₁₋₆ haloalkyl, unless indicated otherwise, means a saturated hydrocarbon radical having up to six carbon atoms, which is substituted with a least one halogen atom, up to perhalo. The radical may be cyclic, linear or branched with single or multiple branching. The halo substituent(s) include fluoro, chloro, bromo, or iodo. Fluoro, chloro and bromo are preferred, and fluoro and chloro are more preferred. The halogen substituent(s) can be located on any available carbon. When more than one halogen substituent is present on this moiety, they may be the same or different. Examples of such halogenated alkyl substituents include but are not limited to chloromethyl, dichloromethyl, trichloromethyl, fluoromethyl, difluoromethyl, trifluoromethyl, 2,2,2-trifluoroethyl, and 1,1,2,2-tetrafluoroethyl, and the like.

The term C₁₋₆ alkoxy, unless indicated otherwise, means a cyclic, straight or branched chain alkoxy group having from one to six saturated carbon atoms which may be cyclic, linear or branched with single or multiple branching, and includes such groups as methoxy, ethoxy, *n-*propoxy, isopropoxy, butoxy, pentoxy and the like. It also includes halogenated groups such as 2, 2-dichloroethoxy, trifluoromethoxy, and the like.

Halo or halogen means fluoro, chloro, bromo, or iodo. Fluoro, chloro and bromo are preferred, and fluoro and chloro are more preferred.

C₁₋₃alkylamine, unless indicated otherwise, means methylamino, ethylamino, propylamino or isopropylamino.

Examples of C₁₋₆dialkylamine include but are not limited to diethylamino, ethyl-isopropylamino, methyl-isobutylamino and dihexylamino.

The term heteroaryl refers to both monocyclic and bicyclic heteroaryl rings. Monocyclic heteroaryl means an aromatic monocyclic ring having 5 to 6 ring atoms and 1-4 hetero atoms selected from N, O and S, the remaining atoms being carbon. When more than one hetero atom is present in the moiety, they are selected independently from the other(s) so that they may be the same or different. Monocyclic heteroaryl rings include, but are not limited to pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, and triazine.

Bicyclic heteroaryl means fused bicyclic moieties where one of the rings is chosen from the monocyclic heteroaryl rings described above and the second ring is either benzene or another monocyclic heteroaryl ring described above. When both rings in the bicyclic moiety are heteroaryl rings, they may be the same or different, as long as they are chemically accessible by means known in the art. Bicyclic heteroaryl rings include synthetically accessible 5-5, 5-6, or 6-6 fused bicyclic aromatic structures including, for example but not by way of limitation, benzoxazole (fused phenyl and oxazole), quinoline (fused phenyl and pyridine), imidazopyrimidine (fused imidazole and pyrimidine), and the like.

Where indicated, the bicyclic heteroaryl moieties may be partially saturated. When partially saturated either the monocyclic heteroaryl ring as described above is fully or partially saturated, the second ring as described above is either fully or partially saturated or both rings are partially saturated.

The term "heterocyclic group", unless indicated otherwise, means monocyclic and bicyclic moieties containing at least one atom selected from oxygen, nitrogen and sulfur, which is saturated or partially saturated, and includes, by no way of limitation, tetrahydropyran, tetrahydrofuran, 1,3-dioxolane, 1,4-dioxane, morpholine, thiomorpholine, piperazine, piperidine, piperidinone, tetrahydropyrimidone, pentamethylene sulfide, tetramethylene sulfide, dihydropyrane, dihydiofuran, dihydrothiophene and the like.

The term "C₁₋₃ alkyl-phenyl" includes, for example, 2-methylphenyl, isopropylphenyl, 3-phenylpropyl, or 2-phenyl-1-methylethyl. Substituted examples include 2-[2-chlorophenyl]ethyl, 3,4-dimethylphenylmethyl, and the like.

Unless otherwise stated or indicated, the term "aryl" includes 6-12 membered mono or bicyclic aromatic hydrocarbon groups (e.g., phenyl, naphthalene, azulene, indene group) having 0, 1, 2, 3, 4, 5 or 6 substituents.

The compounds of formula (I) may contain one or more asymmetric centers, depending upon the location and nature of the various substituents desired. Asymmetric carbon atoms may be present in the (*R*) or (*S*) configuration or (*R*,*S*) configuration. In certain instances, asymmetry may also be present due to restricted rotation about a given bond, for example, the central bond adjoining two substituted aromatic rings of the specified compounds. Substituents on a ring may also be present in either cis or trans form. It is intended that all such configurations (including enantiomers and diastereomers), are included within the scope of the present invention. Preferred compounds are those with the absolute configuration of the compound of formula (I) which produces the more desirable biological activity. Separated, pure or partially purified isomers or racemic mixtures of the compounds of this invention are also included within the scope of the present invention. The purification of said isomers and the separation of said isomeric mixtures can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of formula I can likewise be obtained by chiral syntheses utilizing optically active starting materials.

The present invention also relates to useful forms of the compounds as disclosed herein, such as pharmaceutically acceptable salts, metabolites and prodrugs . The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. Pharmaceutically acceptable salts include those obtained by reacting the main compound, functioning as a base, with an inorganic or organic acid to form a salt, for example, salts of hydrochloric acid, sulfuric acid, phosphoric acid, methane sulfonic acid, camphor sulfonic acid, oxalic acid, maleic acid, succinic acid and citric acid. Pharmaceutically acceptable salts also include those in which the main compound functions as an acid and is reacted with an appropriate base to form, e.g., sodium, potassium, calcium, mangnesium, ammonium, and choline salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts are prepared by reacting the compounds of the invention with the appropriate base via a variety of known methods.

Representative salts of the compounds of this invention include the conventional non-toxic salts and the quaternary ammonium salts which are formed, for example, from inorganic or organic acids or bases by means well known in the art. For example, such acid addition salts include acetate, adipate, alginate, ascorbate, aspartate, benzoate, benzenesulfonate, bisulfate, butyrate, citrate, camphorate, camphorsulfonate, cinnamate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, itaconate, lactate, maleate, mandelate, methanesulfonate, 2-naphthalenesulfonate, nicotinate, nitrate, oxalate, pamoate, pectinate, persulfate, 3-phenylpropionate, picrate, pivalate, propionate, succinate, sulfonate, tartrate, thiocyanate, tosylate, trifluoromethanesulfonate, and undecanoate.

Base salts include alkali metal salts such as potassium and sodium salts, alkaline earth metal salts such as calcium and magnesium salts, and ammonium salts with organic bases such as dicyclohexylamine and N-methyl-D-glucamine. Additionally, basic nitrogen containing groups may be quaternized with such agents as lower alkyl halides such as methyl, ethyl, propyl and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aryl or aralkyl halides like benzyl and phenethyl bromides and others monosubstituted aralkyl halides or polysubstituted aralkyl halides.

Solvates for the purposes of the invention are those forms of the compounds where solvent molecules form a complex in the solid state and include, but are not limited to for example ethanol and methanol. Hydrates are a specific form of solvates, where the solvent molecule is water.

Certain pharmacologically active agents can be further modified with labile functional groups that are cleaved after *in vivo* administration to furnish the parent active agent and the pharmacologically inactive derivatizing group. These derivatives, commonly referred to as prodrugs, can be used, for example, to alter the physicochemical properties of the active agent, to target the active agent to a specific tissue, to alter the pharmacokinetic and pharmacodynamic properties of the active agent, and to reduce undesirable side effects. Prodrugs of the invention include, e.g., the esters of appropriate compounds of this invention that are well-tolerated, pharmaceutically acceptable esters such as alkyl esters including methyl, ethyl, propyl, isopropyl, butyl, isobutyl or pentyl esters. Additional esters such as phenyl-C₁-C₅ alkyl may be used, although methyl ester is preferred.

Methods which can be used to synthesize other prodrugs are described in the following reviews on the subject,
- Higuchi, T.; Stella, V. eds. Prodrugs As Novel Drug Delivery Systems. ACS Symposium Series. American Chemical Society: Washington, DC (1975).
- Roche, E. B. Design of Biopharmaceutical Properties through Prodrugs and Analogs. American Pharmaceutical Association: Washington, DC (1977).
- Sinkula, A. A.; Yalkowsky, S. H. J·Pharm Sci. 1975, 64, 181-210.
- Stella, V. J.; Charman, W. N. Naringrekar, V. H. Drugs 1985, 29, 455-473.
- Bundgaard, H., ed. Design of Prodrugs. Elsevier: New York (1985).
- Stella, V. J.; Himmelstein, K. J. J. Med. Chem. 1980, 23, 1275-1282.
- Han, H-K; Amidon, G. L. AAPS Pharmsci 2000, 2, 1- 11.
- Denny, W. A. Eur. J. Med. Chem. 2001, 36, 577-595.
- Wermuth, C. G. in Wermuth, C. G. ed. The Practice of Medicinal Chemistry Academic Press: San Diego (1996), 697-715.
- Balant, L. P.; Doelker, E. in Wolff, M. E. ed. Burgers Medicinal Chemistry And Drug Discovery John Wiley & Sons: New York (1997), 949-982.

The metabolites of the compounds of this invention include oxidized derivatives of the compounds of formula I, II, X, Z1 and Z2, wherein one or more of the nitrogens are substituted with a hydroxy group; which includes derivatives where the nitrogen atom of the pyridine group is in the oxide form, referred to in the art as 1-oxo-pyridine or has a hydroxy substituent, referred to in the art as 1-hydroxy-pyridine.

### General Preparative Methods

The particular process to be utilized in the preparation of the compounds used in this embodiment of the invention depends upon the specific compound desired. Such factors as the selection of the specific substituents play a role in the path to be followed in the preparation of the specific compounds of this invention. Those factors are readily recognized by one of ordinary skill in the art.

The compounds of the invention may be prepared by use of known chemical redactions and procedures as described in the following published international applications WO 00/42012, WO03/047579, WO 2005/009961, WO 2004/078747 and WO05/000284 and European patent applications EP 04023131.8 and EP 04023130.0.

The compounds of the invention can be made according to conventional chemical methods, and/or as disclosed below, from starting materials which are either commercially available or producible according to routine, conventional chemical methods. General methods for the preparation of the compounds are given below.

The preparation of ureas of formula (I) can be prepared from the condensation of the two arylamine fragments and in the presence of phosgene, di-phosgene, tri-phosgene, carbonyldiimidazole, or equivalents in a solvent that does not react with any of the starting materials, as described in one or more of these published. Alternatively, compounds of formula (I) can be synthesized by reacting amino compounds) with isocyanate compounds as described in one or more of the published international applications described above.

The isocyanates are commercially available or can be synthesized from heterocyclic amines according to methods commonly known to those skilled in the art [e.g. from treatment of an amine with phosgene or a phosgene equivalent such as trichloromethyl chloroformate (diphosgene), bis(trichloromethyl)carbonate (triphosgene), or NN'-carbonyldiimidazole (CDI); or, alternatively by a Curtius-type rearrangement of an amide, or a carboxylic acid derivative, such as an ester, an acid halide or an anhydride].

Aryl amines of formulas are commercially available, or can be synthesized according to methods commonly known to those skilled in the art. Aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. Hydrogenation Methods; Academic Press: London, UK (1985)). Nitroaryls may also be directly reduced using a strong hydride source, such as LiAlH₄ (Seyden-Penne. Reductions by the Alumino- and borohydrides in Organic Synthesis; VCH Publishers: New York (1991)), or using a zero valent metal, such as Fe, Sn or Ca, often in acidic media. Many methods exist for the synthesis of nitroaryls (March. Advanced Organic Chemistry, 3rd Ed.; John Wiley: New York (1985). Larock. Comprehensive Organic Transformations; VCH Publishers: New York (1989)). Nitro aryls are commonly formed by electrophilic aromatic nitration using HNO₃, or an alternative NO₂⁺ source.

Pyridine-1-oxides of formula (I) where the pyridine ring carries a hydroxy substituent on its nitrogen atom, and A, B, L are broadly defined as above can be prepared from the corresponding pyridines using oxidation conditions know in the art. Some examples are as follows:
- peracids such as meta chloroperbenzoic acids in chlorinated solvents such as dichloromethane, dichloroethane, or chloroform (Markgraf et al., Tetrahedron 1991, 47, 183);
- (Me₃SiO)₂ in the presence of a catalytic amount of perrhenic acid in chlorinated solvents such as dichloromethane (Coperet et al., Terahedron Lett. 1998, 39, 761);
- Perfluoro-cis-2-butyl-3-propyloxaziridine in several combinations of halogenated solvents (Amone et al., Tetrahedron 1998, 54, 7831);
- Hypofluoric acid - acetonitrile complex in chloroform (Dayan et al., Synthesis 1999, 1427);
- Oxone, in the presence of a base such as KOH, in water (Robker et al., J. Chem. Res., Synop. 1993, 10, 412);
- Magnesium monoperoxyphthalate, in the presence of glacial acetic acid (Klemm et al., J. Heterocylic Chem. 1990, 6, 1537);
- Hydrogen peroxide, in the presence of water and acetic acid (Lin A.J., Org. Prep. Proced. Int. 1991, 23(1), 114);
- Dimethyldioxirane in acetone (Boyd et al., J. Chem. Soc., Perkin Trans. 1991, 9, 2189).

In addition, specific methods for preparing diaryl ureas and intermediate compounds are already described elsewhere in the patent literature, and can be adapted to the compounds of the present invention. For example, Miller S. et al, "Inhibition of p38 Kinase using Symmetrical and Unsymmetrical Diphenyl Ureas" PCT Int. Appl. WO 99 32463, Miller, S et al. "Inhibition of raf Kinase using Symmetrical and Unsymmetrical Substituted Diphenyl Ureas" PCT Int. Appl.*,* WO 99 32436, Dumas, J. et al., "Inhibition of p38 Kinase Activity using Substituted Heterocyclic Ureas" PCT Int. Appl.*,* WO 99 32111, Dumas, J. et al., "Method for the Treatment of Neoplasm by Inhibition of raf Kinase using N-Heteroaryl-N'-(hetero)arylureas" PCT Int. Appl.*,* WO 99 32106, Dumas, J. et al., "Inhibition of p38 Kinase Activity using Aryl- and Heteroaryl- Substituted Heterocyclic Ureas" PCT Int. Appl.*,* WO 99 32110, Dumas, J., et al., "Inhibition of raf Kinase using Aryl- and Heteroaryl- Substituted Heterocyclic Ureas" PCT Int. Appl.*,* WO 99 32455, Riedl, B., et al., "O-Carboxy Aryl Substituted Diphenyl Ureas as raf Kinase Inhibitors" PCT Int. Appl.*,* WO 00 42012, Riedl, B., et al., "O-Carboxy Aryl Substituted Diphenyl Ureas as p38 Kinase Inhibitors" PCT Int. Appl.*,* WO 00 41698, Dumas, J. et al. "Heteroaryl ureas containing nitrogen hetero-atoms as p38 kinase inhibitors" U.S. Pat. Appl. Publ.*,* US 20020065296, Dumas, J. et al. "Preparation of N-aryl-N'-[(acylphenoxy) phenyl]ureas as raf kinase inhibitors" PCT Int. Appl., WO 02 62763, Dumas, J. et al. "Inhibition of raf kinase using quinolyl, isoquinolyl or pyridyl ureas" PCT Int. Appl.*,* WO 02 85857, Dumas, J. et al. "Preparation of quinolyl, isoquinolyl or pyridyl-ureas as inhibitors of raf kinase for the treatment of tumors and/or cancerous cell growth" U.S. Pat. Appl. Publ., US 20020165394.

Synthetic transformations that may be employed in the synthesis of compounds of formula (I) and in the synthesis of intermediates involved in the synthesis of compounds of formula (I) are known by or accessible to one skilled in the art. Collections of synthetic transformations may be found in compilations, such as:
- J. March. Advanced Organic Chemistry, 4th ed.; John Wiley: New York (1992);
- R.C. Larock. Comprehensive Organic Transformations, 2nd ed.; Wiley-VCH: New York (1999);
- F.A. Carey; R.J. Sundberg. Advanced Organic Chemistry, 2nd ed.; Plenum Press: New York (1984);
- T.W. Greene; P.G.M. Wuts. Protective Groups in Organic Synthesis, 3rd ed.; John Wiley: New York (1999);
- L.S. Hegedus. Transition Metals in the Synthesis of Complex Organic Molecules, 2nd ed.; University Science Books: Mill Valley, CA (1994);
- L.A. Paquette, Ed. The Encyclopedia of Reagents for Organic Synthesis; John Wiley: New York (1994);
- A.R Katritzky; O. Meth-Cohn; C.W. Rees, Eds. Comprehensive Organic Functional Group Transformations; Pergamon Press: Oxford, UK (1995);
- G. Wilkinson; F.G A. Stone; E.W. Abel, Eds. Comprehensive Organometallic Chemistry; Pergamon Press: Oxford, UK (1982);
- B.M. Trost; I. Fleming. Comprehensive Organic Synthesis; Pergamon Press: Oxford, UK (1991);
- A.R. Katritzky; C.W. Rees Eds. Comprehensive Heterocylic Chemistry; Pergamon Press: Oxford, UK (1984);
- A.R. Katritzky; C.W. Rees; E.F.V. Scriven, Eds. Comprehensive Heterocylic Chemistry II; Pergamon Press: Oxford, UK (1996); and
- C. Hansch; P.G. Sammes; J.B. Taylor, Eds. Comprehensive Medicinal Chemistry: Pergamon Press: Oxford, UK (1990).

In addition, recurring reviews of synthetic methodology and related topics include Organic Reactions; John Wiley: New York; Organic Syntheses; John Wiley: New York; Reagents for Organic Synthesis: John Wiley: New York; The Total Synthesis of Natural Products; John Wiley: New York; The Organic Chemistry of Drug Synthesis; John Wiley: New York; Annual Reports in Organic Synthesis; Academic Press: San Diego CA; and Methoden der Organischen Chemie (Houben-Weyl); Thieme: Stuttgart, Germany. Furthermore, databases of synthetic transformations include *Chemical Abstracts*, which may be searched using either CAS OnLine or SciFinder, *Handbuch der Organischen Chemie* (Beilstein), which may be searched using SpotFire, and REACCS.

### Further therapeutic agents

The compounds of formula I according to the .present invention can be combined with further therapeutic agents presently used to treat, prevent or manage pulmonary hypertension such as, but notlimited to, anticoagulants, diuretics, cardiac glycosides, calcium channel blockers, vasodilators, prostacyclin analogues, endothelium antagonists, phosphodiesterase inhibitors, endopeptidase inhibitors, lipid lowering agents, thromboxane inhibitors and other therapeutics known to reduce pulmonary artery pressure.

Examples of anticoagulants include, but are not limited to, e.g. warfarin useful in the treatment of patients with pulmonary hypertension having an increased risk of thrombosis and thromboembolism.

Examples of calcium channel blockers include, but are not limited to, diltiazem, felodipine, amlodipine and nifedipine particularly useful for vasoreactive patients at right heart catheterization.

Examples of vasodilators include, but are not limited to, e.g. prostacyclin, epoprostenol, treprostinil, nitric oxide (NO).

Examples of phosphodiesterase inhibitors include, but are not limited to, particularly phosphodiesterase V inhibitors such as e.g. tadalafil, sildenafil and vardenafil.

Examples of endothelin antagonists include, but are not limited to, e.g. bosentan and sitaxentan, preferably bosetan.

Examples of prostacyclin analogues include, but are not limited to, e.g. ilomedin, treprostinil and epoprostenol.

Examples of lipid lowering agents include, but are not limited to, e.g. HMG CoA reductase inhibitors such as simvastatin, pravastatin, atorvastatin, lovastatin, itavastatin, fluvastatin, pitavastatin, rosuvastatin, ZD-4522 and cerivastatin

Examples diuretics include, but are not limited to, e.g. chlorthalidon, indapamid, bendro-flumethiazid, metolazon, cyclopenthiazid, polythiazid, mefrusid, ximapid, chlorothiazid and hydrochlorothiazid particularly useful to manage peripheral edema.

Examples of other therapeutics known to reduce pulmonary artery pressure include, but are not limited to, e.g. ACE inhibitors such as enalapril, ramipril, captopril, cilazapril, trandolapril, fosinopril, quinapril, moexipril, lisinopril and perindopril, or AT II inhibitors such as losartan, candesartan, irbesartan, embusartan, valsartan and telmisartan, or iloprost, betaprost, L-arginine, omapatrilat, oxygen particularly useful in those patients with resting or exercise-induced hypoxemia or digoxin particularly useful to improve right ventricular function in patients with right ventricular failure.

In addition the compounds and combinations of the invention can furthermore be combined with kinase inhibitors and/or elastase inhibitors.

Examples of kinase inhibitors include, but are not limited to, e.g. BMS-354825, canertinib, erlotinib, gefitinib, imatinib, lapatinib, lestaurtinib, lonafarnib, pegaptanib, pelitinib, semaxanib, tandutinib, tipifarnib, vatalanib, lonidamine, fasudil, leflunomide, bortezomib, imatinib, erlotinib and glivec. Preference is given to glivec.

### Indications

The compounds and combinations according to the present invention can be used for manufacture of a medicament for treating, preventing and managing pulmonary hypertension. Also the present invention provides methods of treating, preventing and managing pulmonary hypertension, comprising administering effective amounts of at least one compound of formula I and optionally at least one further therapeutic agent according to the invention. An "effective amount" is the quantity of the compound that is useful to achieve the desired result, e.g., to treat, prevent or manage the disease or condition.

The term "pulmonary hypertension" according to the invention include, but is not limited to, primary pulmonary hypertension, secondary pulmonary hypertension, familial pulmonary hypertension, sporadic pulmonary hypertension, precapillary pulmonary hypertension, pulmonary arterial, pulmonary artery hypertension, idiopathic pulmonary hypertension, thrombotic pulmonary arteriopathy, plexogenic pulmonary arteriopathy and pulmonary hypertension associated with or related to, left ventricular dysfunction, mitral valvilar disease, constrictivepericarditis, aortic stenosis, cardiomyopathy, mediastinal fibrosis, anomalous pulmonary venous drainage, pulmonary venoocclusive disease, collagen vascular disease, congenital heart disease, congenital heart disease, pulmonary venus hypertension, chronic obstructive pulmonary disease, interstitial lung disease, sleep-disordered breathing, alveolarhyperventilation disorder, chronic exposure to high altitude, neonatal lung disease, alveolar-capillary dysplasia, sickle cell disease, other coagulation disorders, chronic thromboemboli, connective tissue disease, lupus, schistosomiasis, sarcoidosis or pulmonary capillary hemangiomatosis.

Any form of pulmonary hypertension can be treated in accordance with the present invention, including, but not limited to, mild, e.g., associated with increases of mean blood pressure of about 20-30 mm Hg at rest; moderate, e.g., associated with increases of 30-39 mm Hg at rest; and, severe, e.g., associated with increases of 40 mm Hg or more at rest.

Pulmonary hypertension includes pulmonary arterial hypertension (PAH), and includes, primary pulmonary hypertension (PPH), idiopathic PAH (IPAH), familial PAH (FPAH). Several classifications systems for pulmonary hypertension have been published, including the Evian Nomenclature and Classification of pulmonary hypertension (PH) (1998) and the Revised Nomenclature and Classification of PH (2003). See, Lewis et al., Chest, 2004, 126, 73-10, which is hereby incorporated by reference in its entirety. Any disease PH listed in these classification schemes can be treated, managed, or prevented in accordance with the present invention. Risk factors and diagnostic criteria for PH are described in McGoon et al., Chest, 126, 14-34, 2004, .

The following list is the 2003 classification proposed at the Third World Conference on Pulmonary Hypertension: PAH, IPAH, FPAH, collagen vascular disease, congenital systemic to pulmonary shunts (large, small, repaired or nonrepaired), Portal hypertension, drugs and toxins, other (glycogen storage disease, gaucher disease, hereditary hemorrhagic telangiectasia, hemoglobinopathies, myeloproliferative disorders, splenectomy), associated with significant venous or capillary involvement, pulmonary venous hypertension, pulmonary capillary hemangiomatosis, pulmonary venous hypertension, left-sided atrial ventricular heart disease, left-sided valvular heart disease, pulmonary hypertension associated with hypoxemia, COPD, interstitial lung disease, sleep-disordered breathing, alveolar hypoventilation disorders, chronic exposure to high altitude, PH due to chronic thrombotic and/or embolic disease, thromboembolic obstruction of proximal pulmonary arteries, thromboembolic obstruction of distal pulmonary arteries, pulmonary embolism (tumor, parasites, foreign material), sarcoidosis, histiocytosis X, lymphangiomatosis, compression of pulmonary vessels (adenopathy, tumor, fibrosing mediastinitis)

Any of the above-mentioned disorders can be associated with an increased risk of pulmonary hypertension, including, subjects having, e.g., congenital heart disease (e.g., Eisenmenger syndrome); left heart disease; pulmonary venous disease (e.g., fibrosis tissue narrowing or occluding pulmonary veins and venules); pulmonary arterial disease; diseases causing alveolar hypoxia; fibrotic lung diseases; Williams syndrome; subjects with intravenous drug abuse injury; pulmonary vasculitis (such as Wegener's, Goodpasture's, and Churg-Strauss syndromes); emphysema; chronic bronchitis; kyphoscoliosis; cystic fibrosis; obesity-hyper-ventilation and sleep apnea disorders; pulmonary fibrosis; sarcoidosis; silocosis; CREST (calcinosis cutis, Raynaud phenomenon; esophageal motility disorder; sclerodactyly, and teleangiectasia) and other connective tissue diseases. For example, a subject who possesses a BMPR2 mutation (bone morphogenetic protein receptor II) has a 10-20% lifetime risk of acquiring FPAH. Subjects with hereditary hemorrhagic telangiectasa were also identified as being at risk for IPAH, especially those carrying mutations in ALK1. See, McGoon et al., Chest, 2004, 126, 14-34.

According to the invention the term "treating" refers to the administration of a pharmaceutical composition after the onset of symptoms of pulmonary hypertension, whereas "preventing" refers to the administration prior to the onset of symptoms, particularly to patients at risk of pulmonary hypertension. The term "managing" encompasses preventing the recurrence of pulmonary hypertension in a patient who suffered from pulmonary hypertension.

### Administration

Compounds or drug combinations of the present invention can be administered in any form by any effective route, including, e.g., oral, parenteral, enteral, intravenous, intraperitoneal, topical, transdermal (e.g., using any standard patch), ophthalmic, nasally, local, non-oral, such as aerosal, inhalation, subcutaneous, intramuscular, buccal, sublingual, rectal, vaginal, intra-arterial, and intrathecal, etc. They can be administered alone, or in combination with any ingredient(s), active or inactive.

Preference is given to an oral administration.

Compounds or drug combinations of the present invention can be converted in a known manners into the usual formulations, which may be liquid or solid formulations e.g. without limitation normal and enteric coated tablets, capsules, pills, powders, granules, elixirs, tinctures, solution, suspensions, syrups, solid and liquid aerosols and emulsions.

Examples of solid formulations for oral administration are described in US provisional application Nos. 60/605,753 and 60/658,827.

The combinations of the present invention can be administered at any time and in any effective form. For example, the compounds can be administered simultaneously, e.g., as a single composition or dosage unit (e.g., a pill or liquid containing both compositions), or they can be administered as separate compositions, but at the same time (e.g., where one drug is administered intravenously and the other is administered orally or intramuscularly). The drugs can also be administered sequentially at different times. Agents can be formulated conventionally to achieve the desired rates of release over extended period of times, e.g., 12-hours, 24-hours. This can be achieved by using agents and/or their derivatives which have suitable metabolic half-lives, and/or by using controlled release formulations.

The drug combinations can be synergistic, e.g., where the joint action of the drugs is such that the combined effect is greater than the algebraic sum of their individual effects. Thus, reduced amounts of the drugs can be administered, e.g., reducing toxicity or other deleterious or unwanted effects, and/or using the same amounts as used when the agents are administered alone, but achieving greater efficacy.

Compounds or drug combinations of the present invention can be further combined with any other suitable additive or pharmaceutically acceptable carrier. Such additives include any of the substances already mentioned, as well as any of those used conventionally, such as those described in Remington: The Science and Practice of Pharmacy (Gennaro and Gennaro, eds, 20th edition, Lippincott Williams & Wilkins, 2000); Theory and Practice of Industrial Pharmacy (Lachman et al., eds., 3rd edition, Lippincott Williams. & Wilkins, 1986); Encyclopedia of Pharmaceutical Technology (Swarbrick and Boylan, eds., 2nd edition, Marcel Dekker, 2002). These can be referred to herein as "pharmaceutically acceptable carriers" to indicate they are combined with the active drug and can be administered safely to a subject for therapeutic purposes.

In addition, compounds or drug combinations of the present invention can be administered with other active agents or other therapies that are utilized to treat any of the above-mentioned diseases and/or conditions.

Other therapies according to the invention include, but are not limited to, e.g. surgery such as arterial septostomy and lung transplantation therapy. Arterial septostomy and lung transplantation therapy may be necessary for pulmonary hypertension patients who failed to respond to medicinal therapy.

The present invention provides also combinations of at least one compound of Formula I and at least one other therapeutic agent mentioned above useful in treating a disease or disorder. "Combinations" for the purposes of the invention include:
- single compositions or dosage forms which contain at least one compound of Formula I and at least one other therapeutic agent mentioned above;
- combination packs containing at least one compound of Formula I and at least one other therapeutic agent mentioned above to be administered concurrently or sequentially;
- kits which comprise at least one compound of Formula I and at least one other therapeutic agent mentioned above packaged separate from one another as unit dosages or as independent unit dosages, with or without instructions that they be administered concurrently or sequentially; and
- separate independent dosage forms of at least one compound of Formula I and at least one other therapeutic agent mentioned above which cooperate to achieve a therapeutic effect, e.g., treatment of the same disease, when administered concurrently or sequentially.

The dosage of each agent of the combination can be selected with reference to the other and/or the type of disease and/or the disease status in order to provide the desired therapeutic activity. For example, the active agents in the combination can be present and administered in a fixed combination. "Fixed combination" is intended here to mean pharmaceutical forms in which the components are present in a fixed ratio that provides the desired efficacy. These amounts can be determined routinely for a particular patient, where various parameters are utilized to select the appropriate dosage (e.g., type of disease, age of patient, disease status, patient health, weight, etc.), or the amounts can be relatively standard.

The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

Preference is given to an amount of the compound of formula I from 20 to 2000 mg, preferably from 40 to 800 mg, more preferably from 50 to 600 mg.

Particular preference is given to an amount of p-toluenesulfonic acid salt of 4{4-[3-(4-chloro-3-trifluoromethylphenyl)-ureido]-phenoxy}-pyridine-2-carboxylic acid methyl amide in the pharmaceutical composition from 27 to 2740 mg, preferably from 54 to 1096, more preferably from 68 to 822 mg.

In another embodiment of the invention the compound of formula I is administered in combination with at least one further therapeutic agent in an amount that those of ordinary skill in the art can determine by their professional judgement.

The pharmaceutical composition according to the invention is administered one or more, preferably up to three, more preferably up to two times per day. Preference is given to an administration via the oral route. With each administration the number of tablets or capsules taken in at the same time should not exceed two.

Nevertheless, it may in some cases be advantageous to deviate from the amounts specified, depending on body weight, individual behavior toward the active ingredient, type of preparation and time or interval over which the administration is effected. For instance, less than the aforementioned minimum amounts may be sufficient in some cases, while the upper limit specified has to be exceeded in other cases. In the case of administration of relatively large amounts, it may be advisable to divide these into several individual doses over the day.

The combination can comprise effective amounts of at least one compound of Formula I and at least one other therapeutic agent mentioned above, which achieves a greater therapeutic efficacy than when either compound is used alone. The combination can be useful to treat, prevent or manage pulmonary hypertension, where the therapeutic effect is not observed when the agents are used alone, or where an enhanced effect is observed when the combination is administered.

The relative ratios of each compound in the combination can also be selected based on their respective mechanisms of action and the disease biology. The relative ratios of each compound can vary widely and this invention includes combinations for treating, preventing or managing pulmonary hypertension where the amounts of the formula I compound and the other therapeutic agent can be adjusted routinely such that either is present in higher amounts.

The release of one or more agents of the combination can also be controlled, where appropriate, to provide the desired therapeutic activity when in a single dosage form, combination pack, kit or when in separate independent dosage forms.

Preference is given to a combination comprising at least one compound of formula I and at least one compound selected from the group consisting of phosphodiesterase V inhibitors, endothelin antagonists, prostacyclin analogues, kinase inhibitors and elastase inhibitors. More preferably a combination comprising 4{4-[3-(4-chloro-3-trifluoromethylphenyl)-ureido]-phenoxy}-pyridine-2-carboxylic acid methyl amide (BAY 43-9006) or the *p*-toluenesulfonic acid salt of 4{4-[3-(4-chloro-3-trifluoromethylphenyl)-ureido]-phenoxy}-pyridine-2-carboxylic acid methyl amide and at least one compound selected from the group consisting of tadalafil, sildenafil, vardenafil, bosentan, sitaxentan, ilomedin, treprostinil and epoprostenol is used. Most preferably a combination comprising 4{4-[3-(4-chloro-3-trifluoromethylphenyl)-ureido]-phenoxy}-pyridine-2-carboxylic acid methyl amide (BAY 43-9006) or the *p*-toluenesulfonic acid salt of 4{4-[3-(4-chloro-3-trifluoromethylphenyl)-ureido]-phenoxy}-pyridine-2-carboxylic acid methyl amide and bosentan or vardenafil is used.

### Examples:

The effects of the compounds and drug combinations according to the invention are tested in vitro on isolated rat pulmonary arteries and in vivo in monocrotaline-treated rats with pulmonary hypertension.

### Isolated small pulmonary arteries

Male Wistar rats (250-300 g) are anaesthetized with ether, and the lungs are removed. The left pulmonary arterial vessel is dissected and placed in ice-cold Krebs-Henseleit (KH) buffer of following composition (in mmol/1): NaCl 112, KCl 5.9, CaCl₂ 2.0 MgCl₂ 1.2, NaH₂PO₄ 1.2, NaHCO₃ 25, glucose 11.5 and optionally the compound/combination to be tested in a concentration of 10⁻¹⁰ to 10⁻⁴ mol/l.

For measurement of isometric tension, ring segments, 2 mm in length, are mounted in a small vessel chamber myograph. Two wires (40 µm diameter) are introduced through the lumen of the segments and mounted according to the method described by Mulvany and Halpern (Circulation Research 1977; 41:19-26). After a 30 min equilibration period in oxygenated KH solution at 37 °C and pH = 7.4, segments are stretched to their optimal lumen diameter for active tension development which is determined based on the internal circumference-wall tension ratio of the segments by setting their internal circumference to 90% of what the vessels would have if they are exposed to a passive tension equivalent to that produced by a transmural pressure of 30 mmHg.

Afterwards, segments are washed three times with KH solution and left to equilibrate for 30 min. Segment contractility is then tested by an initial exposure to a high K⁺ solution (120 mmol/l K⁺-KH solution, which is identical to KH solution except that NaCl is replaced by KCI on an equimolar basis).

The vessels are than pre-contracted using K⁺ (50 mmol/l) KH solution. When the contraction is stabilized, an accumulative dose response curve of the compound/combination tested is constructed. The stabilized contraction induced by K⁺ (50 mmol/l) KH solution is defined as 100% tension. The relaxation is expressed as percentage tension.

### Pulmonary Artery Pressure in Monocrotaline treated rats

Male Sprague Dawley rats (250-300g) are treated with monocrotaline 60mg/kg subcutanously (=day 0). On day 14 after monocrotaline injection treatment the compound/combination to be tested is administered. On day 28 hemodynamic parameters, i.e. right vetricular pressure, systemic blood pressure, heart rate, arterial and venous oxygen saturation are measured and compared with untreated control animals.

### Results:

The mentioned monocrotaline (MCT) treated rats are randomized to receive the *p*-toluenesulfonic acid salt of 4{4-[3-(4-chloro-3-trifluoromethylphenyl)-ureido]-phenoxy}-pyridine-2-carboxylic acid methyl amide 10 mg/kg or vehicle by gavage once daily after the onset of moderate pulmonary arterial hypertension starting 14 days after the injection of MCT until the final hemodynamic measurement on day 28. In animals with MCT-Induced pulmonary arterial hypertension treatment with the *p*-toluenesulfonic acid salt of 4{4-[3-(4-chloro-3-trifluoromethylphenyl)-ureido]-phenoxy}-pyridine-2-carboxylic acid methyl amide markedly decreases right ventricular systolic pressure, compared to vehicle treated animals (control: 25 ± 0,56 mmHg; *p*-toluenesulfonic acid salt of 4{4-[3-(4-chloro-3-trifluoromethylphenyl)-ureido]-phenoxy}-pyridine-2-carboxylic acid methyl amide: 36,50 ± 1,50 mmHg vs. placebo: 71,02 ± 5,38 mmHg) (mean ± SEM). This effect of the *p*-toluenesulfonic acid salt of 4{4-[3-(4-chloro-3-trifluoromethylphenyl)-ureido]-phenoxy}-pyridine-2-carboxylic acid methyl amide is paralleled by a complete inhibition of right ventricular hypertrophy (right ventricle/left ventricle +septum ratio control: 0,26 ± 0,01; the *p*-toluenesulfonic acid salt of 4{4-[3-(4-chloro-3-trifluoromethylphenyl)-ureido]-phenoxy}-pyridine-2-carboxylic acid methyl amide: 0,26 ± 0,01 vs. placebo: 0,54 ± 0,04).

### Example 1: Immediate release tablet and optionally subsequent film-coating

### 1.1 Composition of tablets containing the p-toluenesulfonic acid salt of 4{4-[3-(4-chloro-3-trifluoromethylphenyl)-ureido]-phenoxy}-pyridine-2-carboxylic acid methyl amide

| Composition (mg/tablet) | Tablet A 50 mg | Tablet B 200 mg | Tablet C 200 mg | Tablet D 400 mg |
|---|---|---|---|---|
| ***Tablet core:*** | step a), b) | step a), b), c) ii | Step a), b) c) i | Step a), b) c) i |
| Tosylate salt of compound (I) micronized | 68.5 mg | 274.0 mg | 274.0 mg | 548.0 mg |
| Microcrystalline cellulose | 4.0 mg | 16.0 mg | 16.0 mg | 32.0 mg |
| Croscarmellose sodium | 9.1 mg | 36.4 mg | 36.4 mg | 72.8 mg |
| Hypromellose (5 cP) | 2.55 mg | 10.2 mg | 10.2 mg | 20.4 mg |
| Magnesium stearate | 0.425 mg | 1.7 mg | 2.55 mg^{#1} | 5.10 mg |
| | | | (1.70 - 2.55 mg) | |
| Sodium lauryl sulfate | 0.425 mg | 1.7 mg | 1.7 mg | 3.4 mg |
| ***Weight*** | **85.0 mg** | **340.0 mg** | **340.85 mg** | **681.70 mg** |
| | | | (340.0 - 340.85 mg) | |
| | | | | |
| ***Film-coating:*** | | | | |
| Opadry Red YS2-15531^{#3} | ----- | 10.0 mg | --^{#2}-- | --^{#2}-- |
| Hypromellose (15 cP) | ----- | ----- | 6.00 mg | 9.0 mg |
| | | | (4.8 - 7.2 mg) | (7.2-10.8 mg) |
| Macrogol | 3350----- | ----- | 2.00 mg | 3.0 mg |
| (polyethylene gycol) | | | (1.6 - 2.4 mg) | (2.4-3.6 mg) |
| Titanium dioxide | ----- | ----- | 1.73 mg | 1.6 mg |
| | | | (1.384 - 2.076 mg) | (1.28-1.92 mg) |
| Ferric oxide (red) | ----- | ----- | 0.27 mg | ----- |
| | | | (0.216 - 0.324 mg) | |
| Ferric oxide (yellow) | ----- | ----- | ----- | 1.4 mg |
| | | | | (1.12-1.68 mg) |
| ***Weight of film coat*** | ----- | 10.0 mg | 10.0 mg | 15.0 mg |
| | | | (8.0-12.0 mg) | (12.0 - 18.0 mg) |
| ***Total tablet weight*** | **85.0 mg** | **350.0 mg** | **350.85 mg** | **696.7 mg** |
| | | | (348 - 352.85 mg) | (348.0-352.85 mg) |
| | | | | |
| ***Tablet format*** | **Round** | **round** | **round** | **oval** |
| ***Dimensions of the tablet*** | **diameter: 6 mm** | **diameter: 10 height: 4.5 mm** | **mm,diameter: 10 (±0.3)height: 4.5 (±0.3)** | **mm,length: 18 mm, mm width: 8 mm** |

| | | | | |
|---|---|---|---|---|
| ^{#1} Range for Mg stearate may apply according to manufacturing conditions ^{#2} Range for film coat may apply according to manufacturing conditions Fixed ratio of coating components 60 % (hypromellose) - 20 % (polyethylene glycol) - 17.3 % (titanium dioxide) - 2.7 % ferric oxide ^{#3} Opadry Red YS-15531 ready to use commercial coating system. | | | | |

### 1.2 Process for manufacturing

### Step a) Granulation

4{4-[3-(4-chloro-3-trifluoromethylphenyl)-ureido]-phenoxy}-pyridine-2-carboxylic acid methyl amide micronized, microcrystalline cellulose, croscarmellose sodium, and hypromellose are mixed for 2 minutes in a high shear mixer in order to obtain a powder blend. Sodium lauryl sulfate is dissolved in water. The powder blend is granulated with the solution in a wet granulation process using a high-shear mixer. The granulation process is finished when the granulate achieves a "snow ball like consistency". The wet granulation mass is sized using a 4 mm rasp and then dried in a fluidized bed dryer at an inlet air temperature of 80 - 100 °C until a residual moisture of 0.3 up to 0.7% by weight (loss on drying) is reached. The dry granules are sieved using a 2 mm sieve size.

### Step b) Tablet compression

The granulate is blended with magnesium stearate and croscarmellose sodium using a tumbler blender for from 5 to 10 minutes. The blend is subdivided into single units and compressed to tablets using a standard rotary tablet press at typical tabletting speeds of from 25,000 to 250,000 tablets / hour.

### Step c) Film-coating

### Alternative i:

Hypromellose, polyethylene glycol (Macrogol), titanium dioxide and ferric oxide red are combined with purified water to result in a homogenous coating suspension which is sprayed on the tablets in a perforated drum coater.

### Alternative ii:

The commercially available Opadry Red YS-15531 is combined with purified water to result in a homogenous coating suspension which is sprayed on the tablets in a perforated drum coater.

## Claims

1. Use of a compound of formula I or a pharmaceutically acceptable salt, polymorph, solvate, hydrate, metabolite, prodrug or diastereoisomeric form thereof, for manufacture of a medicament for treating, preventing or managing of pulmonary hypertension,
wherein said compound of formula I is: 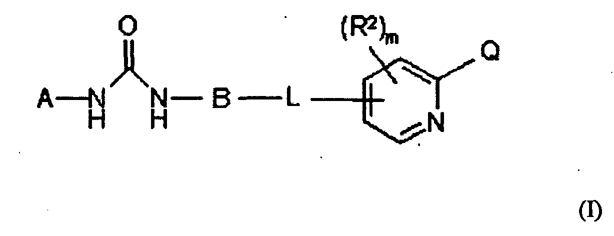 wherein
Q is -C(O)Rₓ
Rₓ is hydroxy, C₁₋₄ alkyl, C₁₋₄ alkoxy or NRₐR_{b},
Rₐ and R_{b} are independently :
a) hydrogen;
b) C₁₋₄alkyl, optionally substituted by
- hydroxy,
- C₁₋₄ alkoxy,
- a heteroaryl group selected from pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, triazine, benzoxazole, isoquioline, quinolines and imidazopyrimidine
- a heterocyclic group selected from tetrahydropyran, tetrahydrofuran, 1,3-dioxolane, 1,4-dioxane, morpholine, thiomorpholine, piperazine, piperidine, piperidinone, tetrahydropyrimidone, pentamethylene sulfide, tetramethylene sulfide, dihydropyrane, dihydrofuran, and dihydrothiophene,
- amino,-NH₂, optionally substituted by one or two C₁₋₄ alkyl groups, or
- phenyl,
c) phenyl optionally substituted with
- halogen, or
- amino,-NH₂, optionally substituted by one or two C₁₋₄ alkyl, or
d)
- a heteroaryl group selected from pyrrole, furan, thiophene,imidazole, pyrazole, thiazole, oxazole, isoxazole, isothiazole, triazole, tetrazole, thiadiazole, oxadiazole, pyridine, pyrimidine, pyridazine, pyrazine, triazine, benzoxazole, isoquioline, quinoline and imidazopyrimidine;
A is an optionally substituted phenyl group of formula 1xx: 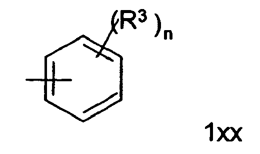 an optionally substituted pyridinyl group of formula 1x: 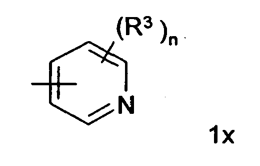 or an optionally substituted naphthyl moiety of formula 1y: 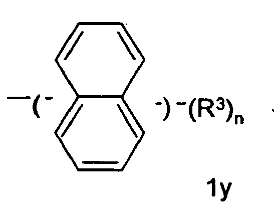 B is optionally substituted phenyl or naphthyl of formulas 2a and 2b: 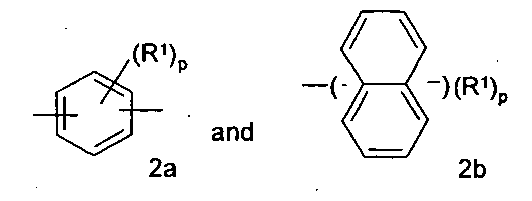 L is a bridging group which is -S- or -O-,
p is 0, 1, 2, 3, or 4,
n is 0, 1, 2, 3, 4, 5 or 6,
m is 0, 1, 2 or 3,
each R¹ is independently: halogen, C₁₋₅ haloalkyl, NO₂, C(O)NR⁴R⁵, C₁₋₆ alkyl, C₁₋₆ dialkylamine, C₁₋₃ alkylamine, CN, amino, hydroxy or C₁₋₃ alkoxy.
each R² is independently: C₁₋₅ alkyl, C₁₋₅ haloalkyl, C₁₋₃ alkoxy, N-oxo or N-hydroxy, each R³ is independently: halogen, R⁴, OR⁴, S(O)R⁴, C(O)R⁴, C(O)NR⁴R⁵, oxo, cyano or nitro (NO₂) and
R⁴ and R⁵ are independently hydrogen, C₁₋₆ alkyl, or up to per-halogenated C₁₋₆ alkyl.

2. The use of claim 1 wherein
A is 3-tert butyl phenyl, 5-tert butyl-2-methoxyphenyl , 5-(trifluoromethyl)-2 phenyl, 3-(trifluoromethyl) -4 chlorophenyl, 3-(trifluoromethyl)-4-bromophenyl or 5- (trifluoromethyl)-4-chloro-2 methoxyphenyl ;
B is 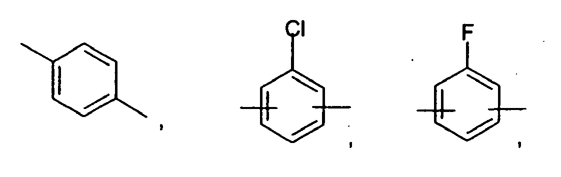 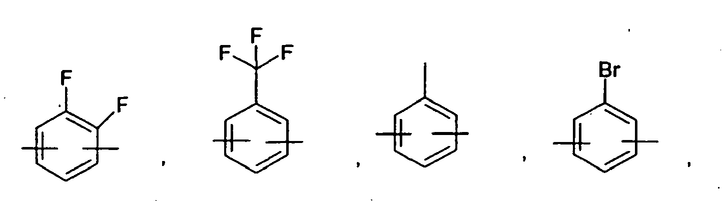 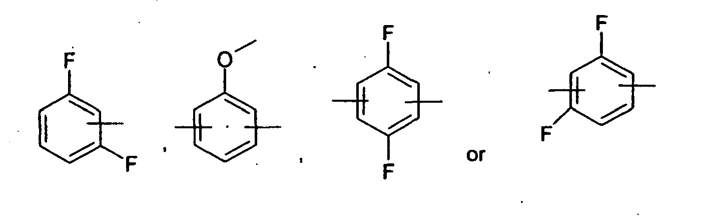
R¹ is fluorine, chorine, bromine, methyl, NO₂, C(O)NH₂, methoxy, SCH₃, trifluoromethyl, or methanesulfonyl;
R² is methyl, ethyl, propyl, oxygen, or cyano and
R³ is trifluoromethyl, methyl, ethyl, propyl, butyl, isopropyl, tert-butyl, chlorine, fluorine, bromine, cyano, methoxy, acetyl, trifluoromethanesulfonyl, trifluoromethoxy, or trifluoromethylthio.

3. The use of any of claims 1 to 2 wherein the compound of formula I is also of formula II below or salts, polymorphs, solvates, hydrates, metabolites, prodrugs or diastereoisomeric forms thereof: 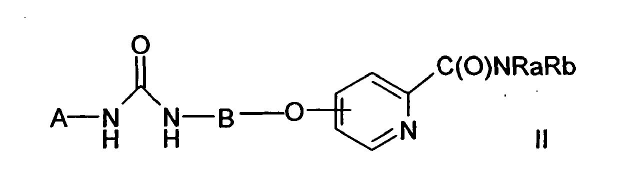 wherein
Ra and Rb are independently hydrogen and C₁-C₄ alkyl,
B of formula II is 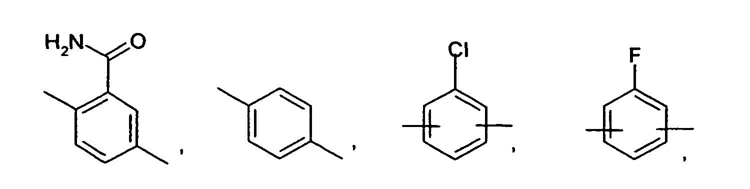 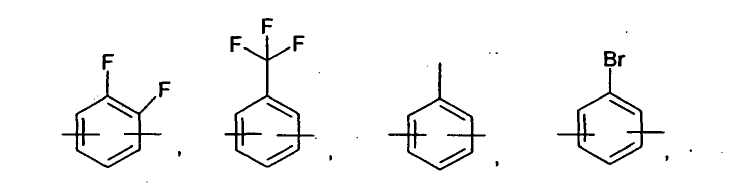 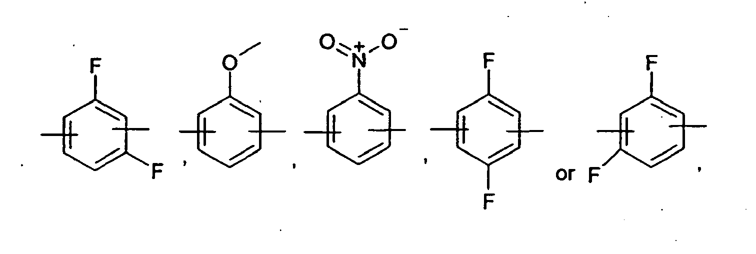 wherein the urea group, -NH-C(O)-NH-, and the oxygen bridging group are not bound to contiguous ring carbons of B, but rather have 1 or 2 ring carbons separating them, and
A of formula (II) is 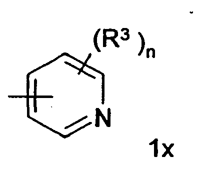 or 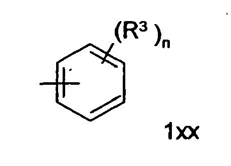 wherein the variable n is 0, 1, 2, 3 or 4, and
R³ is trifluoromethyl, methyl, ethyl, propyl, butyl, isopropyl, tert-butyl, chlorine, fluorine, bromine, cyano, methoxy, acetyl, trifluoromethanesulfonyl, trifluoro- methoxy, or trifluoromethylthio.

4. The use of any of claims 1 to 3 wherein, each R³ substituent is chlorine, trifluoromethyl, tert-butyl or methoxy,
A of formula II is 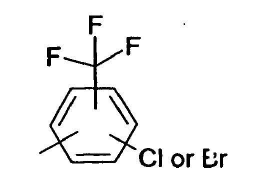 and
B of formula II is phenylene, fluoro substituted phenylene or difluoro substituted phenylene.

5. The use of any of claims 1 to 4 wherein the compound of formula I is also of formula X below or salts, polymorphs, solvates, hydrates, metabolites, prodrugs or diastereoisomeric forms thereof: 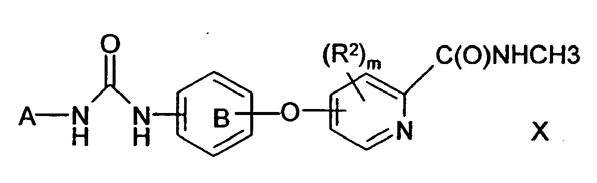 wherein phenyl ring "B" optionally has one halogen substituent, A is an optionally substituted phenyl group of formula 1xx: 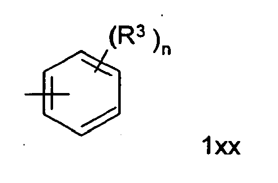 an optionally substituted pyridinyl group of formula 1x: 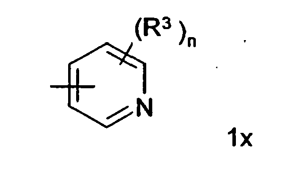 or an optionally substituted naphthyl moiety of formula 1y: 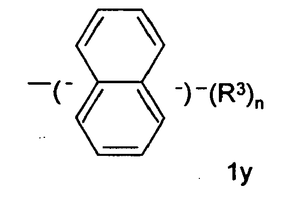 n is 0, 1, 2, 3, 4, 5 or 6,
m is 0,1,2 or 3,
each R² is independently: C₁₋₅ alkyl, C₁₋₅ haloalkyl, C₁₋₃ alkoxy, N-oxo or N-hydroxy,
each R³ is independently: halogen, R⁴, OR⁴, S(O)R⁴, C(O)R⁴, C(O)NR⁴R⁵, oxo, cyano or nitro (NO₂) and
R⁴ and R⁵ are independently hydrogen, C₁₋₆ alkyl, or up to per-halogenated C₁₋₆ alkyl.

6. The use of claim 5 wherein m is zero and A is substituted phenyl with at least one substituent R³.

7. The use of claim 6 wherein R³ is halogen, trifluoromethyl and/or methoxy.

8. The use of claim 1 wherein the compound of formula I also has the structure of one of formulas Z1 or Z2 below or a salt, polymorph, solvate, hydrate, metabolite, prodrug or diastereoisomeric form thereof: 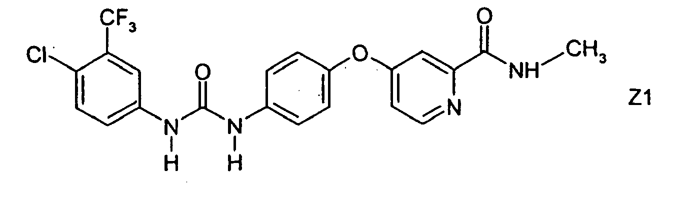 or

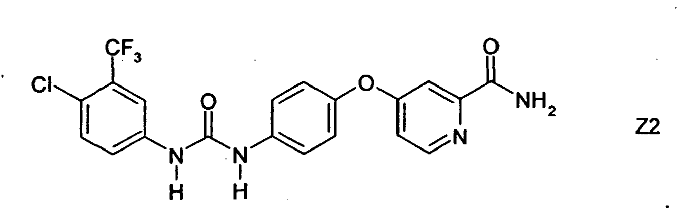

9. The use of claim 8 wherein the compound of formula I is the tosylate salt of the compound of formula Z1.

10. Combination comprising at least one compound of formula I as defined in any of claims 1 to 9 and at least one elastase inhibitor and/or one kinase inhibitor.

11. Combination of claim of 10 wherein the kinase inhibitor is glivec.

12. Combination comprising at least one compound of formula I as defined in any of claims 1 to 9 or a combination as defined in any of claims 10 to 11 and at least one therapeutic agent selected from the group consisting of anticoagulants, diuretics, cardiac glycosides, calcium channel blockers, vasodilators, prostacyclin analogues, endothelium antagonists, phosphodiesterase inhibitors, endopeptidase inhibitors, lipid lowering agents, thromboxane inhibitors and other therapeutics known to reduce pulmonary artery pressure.

13. Combination of claim 12 wherein the further therapeutic agent is a phosphodiesterase V inhibitor, endothelin antagonist or prostacyclin analogue.

14. Combination of claim 12 wherein the further therapeutic agent is tadalafil, sildenafil, vardenafil, bosentan, sitaxentan, ilomedin, treprostinil and epoprostenol.

15. Use of the combination of any of claims 10 to 14 for manufacture of a medicament for treating, preventing or managing of pulmonary hypertension.

16. Pharmaceutical composition comprising a combination as defined in any of claims 10 to 14.

17. Pharmaceutical composition of claim 16 for the treatment of pulmonary hypertension.

## Patentansprüche

1. Verwendung einer Verbindung der Formel I oder eines pharmazeutisch verträglichen Salzes, Polymorphs, Solvats, Hydrats, Metaboliten, Prodrugs oder einer diastereoisomeren Form davon für die Herstellung eines Medikaments zur Behandlung, Vorbeugung oder zum Management von Lungenhochdruck,
wobei die Verbindung der Formel I 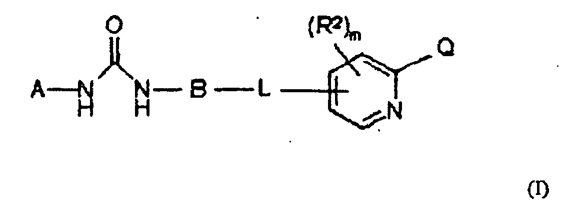 ist,
wobei
Q -C(O)Rₓ ist,
Rₓ Hydroxy, C₁₋₄-Alkyl, C₁₋₄-Alkoxy oder NRₐR_{b} ist,
Rₐ und R_{b} unabhängig
a) Wasserstoff;
b) C₁₋₄-Alkyl, gegebenenfalls substituiert mit
- Hydroxy
- C₁₋₄-Alkoxy,
- einem Heteroarylrest, ausgewählt aus Pyrrol, Furan, Thiophen, Imidazol, Pyrazol, Thiazol, Oxazol, Isoxazol, Isothiazol, Triazol, Tetrazol, Thiadiazol, Oxadiazol, Pyridin, Pyrimidin, Pyridazin, Pyrazin, Triazin, Benzoxazol, Isochiolin, Chinolinen und Imidazopyrimidin,
- einem heterocyclischen Rest, ausgewählt aus Tetrahydropyran, Tetrahydrofuran, 1,3-Dioxolan, 1,4-Dioxan, Morpholin, Thiomorpholin, Piperazin, Piperidin, Piperidinon, Tetrahydropyrimidon, Pentamethylensulfid, Tetramethylensulfid, Dihydropyran, Dihydrofuran und Dihydrothiophen,
- Amino, -NH₂, gegebenenfalls substituiert mit einem oder zwei C₁₋₄-Alkylresten, oder
- Phenyl;
c) Phenyl, gegebenenfalls substituiert mit
- Halogen oder
- Amino, -NH₂, gegebenenfalls substituiert mit einem oder zwei C₁₋₄-Alkyl; oder
d) ein Heteroarylrest, ausgewählt aus Pyrrol, Furan, Thiophen, Imidazol, Pyrazol, Thiazol, Oxazol, Isoxazol, Isothiazol, Triazol, Tetrazol, Thiadiazol, Oxadiazol, Pyridin, Pyrimidin, Pyridazin, Pyrazin, Triazin, Benzoxazol, Isochiolin, Chinolin und Imidazopyrimidin;
sind;
A eine gegebenenfalls substituierte Phenylgruppe der Formel 1xx, 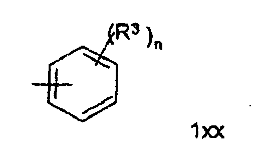 eine gegebenenfalls substituierte Pyridinylgruppe der Formel 1x, 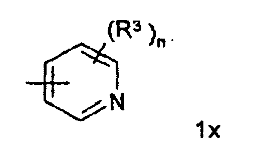 oder eine gegebenenfalls substituierte Naphthyleinheit der Formel 1y 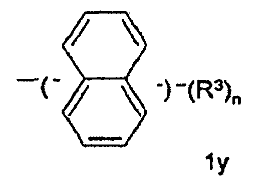 ist,
B gegebenenfalls substituiertes Phenyl oder Naphthyl der Formeln 2a und 2b ist, 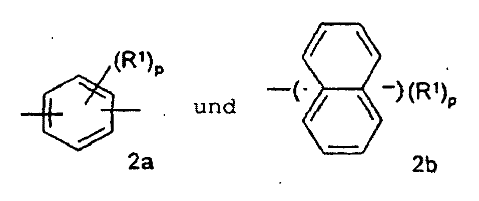 L eine verbrückende Gruppe ist, die -S- oder -O- ist,
p 0, 1, 2, 3 oder 4 ist,
n 0, 1, 2, 3, 4, 5 oder 6 ist,
m 0, 1, 2 oder 3 ist,
jedes R¹ unabhängig Halogen, C₁₋₅-Halogenalkyl, NO₂, C(O)NR⁴R⁵, C₁₋₆-Alkyl, C₁₋₆-Dialkylamin, C₁₋₃-Alkylamin, CN, Amino, Hydroxy oder C₁₋₃-Alkoxy ist,
jedes R² unabhängig C₁₋₅-Alkyl, C₁₋₅-Halogenalkyl, C₁₋₃-Alkoxy, N-Oxo oder N-Hydroxy ist,
jedes R³ unabhängig Halogen, R⁴, OR⁴, S(O)R⁴, C(O)R⁴, C(O)NR⁴R⁵, Oxo, Cyano oder Nitro (NO₂) ist, und
R⁴ und R⁵ unabhängig Wasserstoff, C₁₋₆-Alkyl oder bis zu perhalogeniertes C₁₋₆-Alkyl sind.

2. Verwendung gemäß Anspruch 1, wobei
A 3-tert-Butylphenyl, 5-tert-Butyl-2-methoxyphenyl, 5-(Trifluormethyl)-2-phenyl, 3-(Trifluormethyl)-4- chlorphenyl, 3-(Trifluormethyl)-4-bromphenyl oder 5-(Trifluormethyl)-4-chlor-2-methoxyphenyl ist;
B 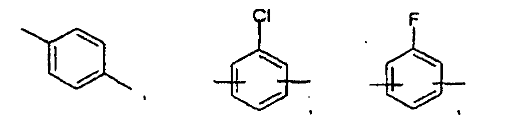 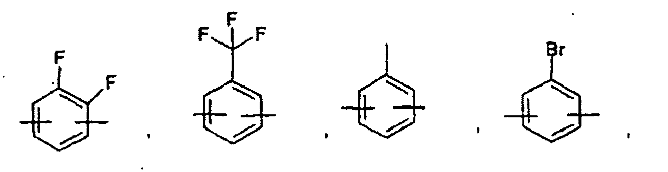 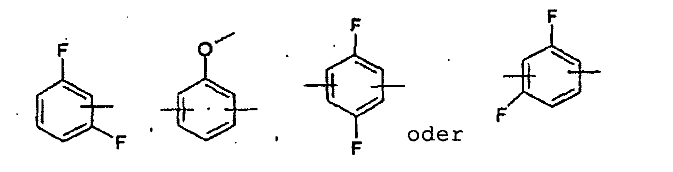 ist;
R¹ Fluor, Chor, Brom, Methyl, NO₂, C(O)NH₂, Methoxy, SCH₃, Trifluormethyl oder Methansulfonyl ist;
R² Methyl, Ethyl, Propyl, Sauerstoff oder Cyano ist;
und
R³ Trifluormethyl, Methyl, Ethyl, Propyl, Butyl, Isopropyl, tert-Butyl, Chlor, Fluor, Brom, Cyano, Methoxy, Acetyl, Trifluormethansulfonyl, Trifluormethoxy oder Trifluormethylthio ist.

3. Verwendung gemäß einem der Ansprüche 1 bis 2, wobei die Verbindung der Formel I auch von der nachstehenden Formel II ist, oder Salze, Polymorphe, Solvate, Hydrate, Metabolite, Prodrugs oder diastereoisomere Formen davon, 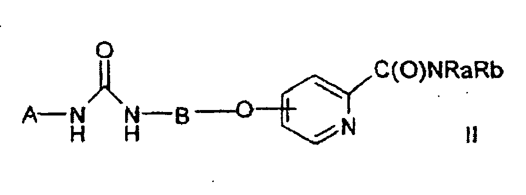 wobei
Ra und Rb unabhängig Wasserstoff oder C₁-C₄-Alkyl sind,
B in der Formel II 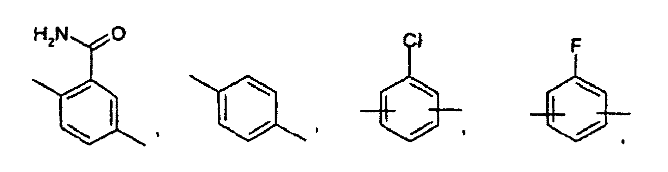 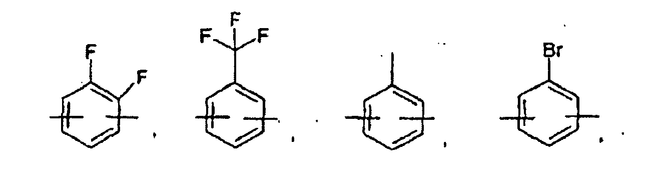 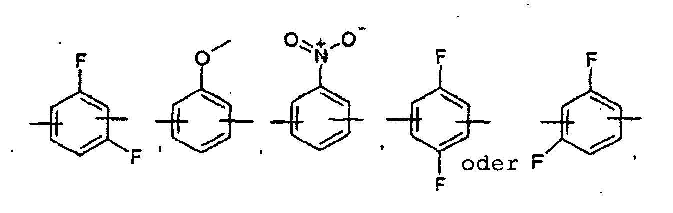 ist,
wobei die Harnstoffgruppe -NH-C(O)-NH- und die verbrückende Sauerstoffgruppe nicht an benachbarte Kohlenstoff-Ringatome von B gebunden sind, sondern 1 oder 2 dazwischen liegende Kohlenstoff-Ringatome aufweisen, und
A in der Formel (II) 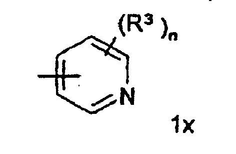 oder 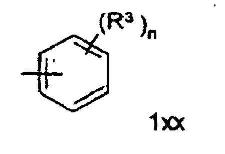 ist,
wobei die Variable n 0, 1, 2, 3 oder 4 ist, und
R³ Trifluormethyl, Methyl, Ethyl, Propyl, Butyl, Isopropyl, tert-Butyl, Chlor, Fluor, Brom, Cyano, Methoxy, Acetyl, Trifluormethansulfonyl, Trifluormethoxy oder Trifluormethylthio ist.

4. Verwendung gemäß einem der Ansprüche 1 bis 3, wobei jeder Substituent R³ Chlor, Trifluormethyl, tert-Butyl oder Methoxy ist,
A in der Formel II 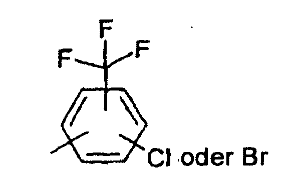 ist, und
B in der Formel II Phenylen, Fluor-substituiertes Phenylen oder Difluor-substituiertes Phenylen ist.

5. Verwendung gemäß einem der Ansprüche 1 bis 4, wobei die Verbindung der Formel I auch von der nachstehenden Formel X ist, oder Salze, Polymorphe, Solvate, Hydrate, Metabolite, Prodrugs oder diastereoisomere Formen davon: 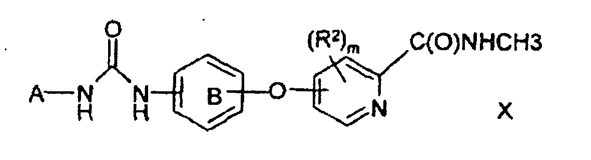 wobei der Phenylring "B" gegebenenfalls einen Halogensubstituenten aufweist,
A eine gegebenenfalls substituierte Phenylgruppe der Formel 1xx, 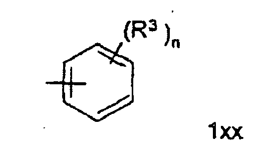 eine gegebenenfalls substituierte Pyridylgruppe der Formel 1x, 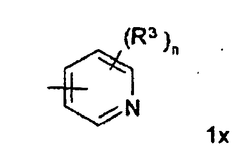 oder eine gegebenenfalls substituierte Naphthyleinheit der Formel 1y 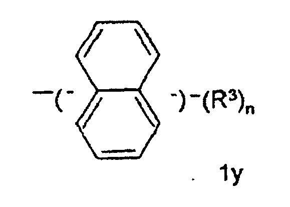 ist,
n 0, 1, 2, 3, 4, 5 oder 6 ist,
m 0, 1, 2 oder 3 ist,
jedes R² unabhängig C₁₋₅-Alkyl, C₁₋₅-Halogenalkyl, C₁₋₃-Alkoxy, N-Oxo oder N-Hydroxy ist,
jedes R³ unabhängig Halogen, R⁴, OR⁴, S (O) R⁴, C (O) R⁴, C (O) NR⁴R⁵, Oxo, Cyano oder Nitro (NO₂) ist, und
R⁴ und R⁵ unabhängig Wasserstoff, C₁₋₆-Alkyl oder bis zu perhalogeniertes C₁₋₆-Alkyl sind.

6. Verwendung gemäß Anspruch 5, wobei m null ist und A substituiertes Phenyl mit wenigstens einem Substituenten R³ ist.

7. Verwendung gemäß Anspruch 6, wobei R³ Halogen, Trifluormethyl und/oder Methoxy ist.

8. Verwendung gemäß Anspruch 1, wobei die Verbindung der Formel I auch die Struktur einer der nachstehenden Formeln Z1 oder Z2 aufweist, oder ein Salz, Polymorph, Solvat, Hydrat, Metabolit, Prodrug oder eine diastereomere Form davon ist: 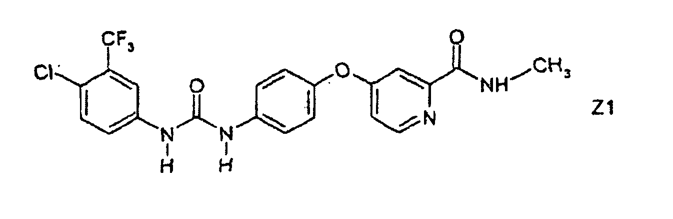 oder

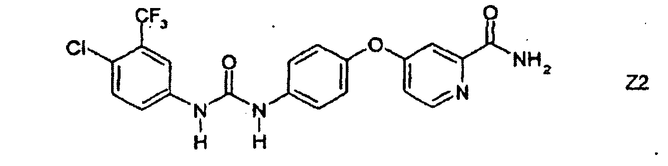

9. Verwendung gemäß Anspruch 8, wobei die Verbindung der Formel I das Tosylatsalz der Verbindung der Formel Z1 ist.

10. Kombination, umfassend wenigstens eine Verbindung der Formel I gemäß einem der Ansprüche 1 bis 9 und wenigstens einen Elastasehemmer und/oder einen Kinasehemmer.

11. Kombination gemäß Anspruch 10, wobei der Kinasehemmer Glivec ist.

12. Kombination, umfassend wenigstens eine Verbindung der Formel I gemäß einem der Ansprüche 1 bis 9 oder eine Kombination gemäß einem der Ansprüche 10 bis 11 und wenigstens ein therapeutisches Mittel, ausgewählt aus der Gruppe, bestehend aus Antikoagulationsmitteln, Diuretika, Herzglykosiden, Calciumkanalblockern, Vasodilatoren, Prostacyclin-Analoga, Endothel-Antagonisten, Phosphodiesterasehemmern, Endopeptidasehemmern, Lipidsenkern, Thromboxanhemmern und anderen Therapeutika, von denen bekannt ist, dass sie den pulmoarteriellen Druck absenken.

13. Kombination gemäß Anspruch 12, wobei das weitere therapeutische Mittel ein Phosphodiesterase-V-Hemmer, Endothelin-Antagonist oder Prostacyclin-Analogon ist.

14. Kombination gemäß Anspruch 12, wobei das weitere therapeutische Mittel Tadalafil, Sildenafil, Vardenafil, Bosentan, Sitaxentan, Ilomedin, Treprostinil oder Epoprostenol ist.

15. Verwendung der Kombination gemäß einem der Ansprüche 10 bis 14 für die Herstellung eines Medikaments zur Behandlung, Vorbeugung oder zum Management von Lungenhochdruck.

16. Pharmazeutische Zusammensetzung, umfassend eine Kombination gemäß einem der Ansprüche 10 bis 14.

17. Pharmazeutische Zusammensetzung gemäß Anspruch 16 für die Behandlung von Lungenhochdruck.

## Revendications

1. Emploi d'un composé de formule I ou de l'un de ses sels, formes polymorphiques, solvates, hydrates, métabolites, promédicaments ou diastéréoisomères de qualité pharmaceutique, dans la fabrication d'un médicament destiné au traitement prophylactique ou thérapeutique ou à la gestion de l'hypertension pulmonaire,
ledit composé de formule I étant le suivant : 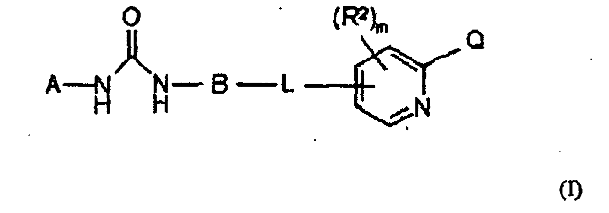 où
Q représente -C(O)Rₓ
Rₓ représente un groupement hydroxy, alkyle en C₁₋₄, alkoxy en C₁₋₄ ou NRₐR_{b}, Rₐ et R_{b} représentant indépendamment :
a) un atome d'hydrogène ;
b) un groupement alkyle en C₁₋₄, éventuellement substitué par
- un groupement hydroxy,
- un groupement alkoxy en C₁₋₄,
- un groupement hétéroaryle choisi parmi les suivants :
pyrrole, furanne, thiophène, imidazole, pyrazole, thiazole, oxazole, isoxazole, isothiazole, triazole, tétrazole, thiadiazole, oxadiazole, pyridine, pyrimidine, pyridazine, pyrazine, triazine, benzoxazole, isoquinoléine, quinoléines et imidazopyrimidine
- un groupement hétérocyclique choisi parmi les suivants : tétrahydropyranne, tétrahydrofuranne, 1,3-dioxolanne, 1,4-dioxanne, morpholine, thiomorpholine, pipérazine, pipéridine, pipéridinone, tétrahydropyrimidone, sulfure de pentaméthylène, sulfure de tétraméthylène, dihydropyranne, dihydrofuranne et dihydrothiophène,
- un groupement amino, -NH₂, éventuellement substitué par un ou deux groupements alkyle en C₁₋₄, ou
- un groupement phényle,
c) un groupement phényle éventuellement substitué par
- un atome d'halogène, ou
- un groupement amino, -NH₂, éventuellement substitué par un ou deux groupements alkyle en C₁₋₄, ou
d)
- un groupement hétéroaryle choisi parmi les suivants : pyrrole, furanne, thiophène, imidazole, pyrazole, thiazole, oxazole, isoxazole, isothiazole, triazole, tétrazole, thiadiazole, oxadiazole, pyridine, pyrimidine, pyridazine, pyrazine, triazine, benzoxazole, isoquinoléine, quinoléine et imidazopyrimidine ;
A représente un groupement phényle éventuellement substitué de formule 1xx : 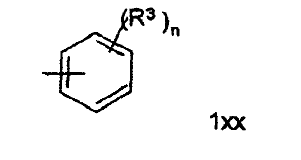 un groupement pyridinyle éventuellement substitué de formule 1x : 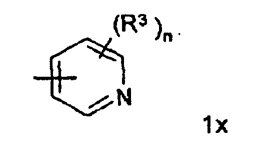 ou un groupement naphtyle éventuellement substitué de formule 1y : 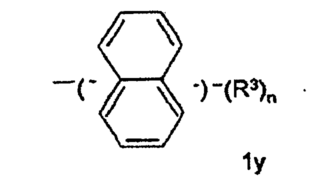 B représente un groupement phényle ou naphtyle éventuellement substitué de formule 2a ou 2b : 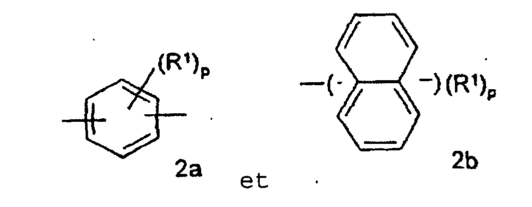 L représente un groupement pontant qui est -S- ou -O-,
p est égal à 0, 1, 2, 3 ou 4,
n est égal à 0, 1, 2, 3, 4, 5 ou 6,
m est égal à 0, 1, 2 ou 3,
chacun des groupements R¹ représente indépendamment : un atome d'halogène ou un groupement halogénoalkyle en C₁₋₅, NO₂, C (O) NR⁴R⁵, alkyle en C₁₋₆, di- (alkyle en C₁₋₆)-amine, (alkyle en C₁₋₃) -amine, CN, amino, hydroxy ou alkoxy en C₁₋₃,
chacun des groupements R² représente indépendamment : un groupement alkyle en C₁₋₅, halogénoalkyle en C₁₋₅, alkoxy en C₁₋₃, N-oxo ou N-hydroxy,
chacun des groupements R³ représente indépendamment : un atome d'halogène ou un groupement R⁴ , OR⁴ , S (O) R⁴, C (O) R⁴, C (O) NR⁴R⁵, oxo, cyano ou nitro (NO₂) et
R⁴ et R⁵ représentent indépendamment un atome d'hydrogène ou un groupement alkyle en C₁₋₆, ou un groupement alkyle en C₁₋₆ substitué par des atomes d'halogène, éventuellement perhalogéné.

2. Emploi conforme à la revendication 1, où
A représente un groupement 3-tert-butylphényle, 5-tert-butyl-2-méthoxyphényle, 5-(trifluorométhyl)-2-phényle, 3-(trifluorométhyl)-4-chlorophényle, 3-(trifluorométhyl)-4-bromophényle ou 5-(trifluorométhyl)-4-chloro-2 méthoxyphényle ;
B représente 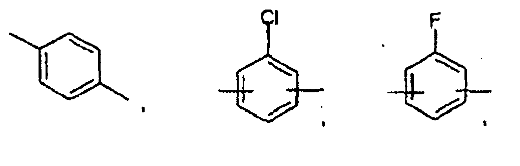 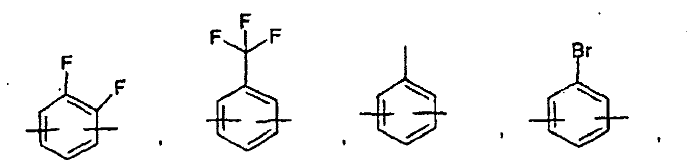 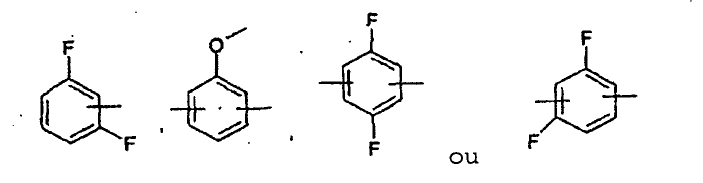 R¹ représente un atome de fluor, de chlore ou de brome ou un groupement méthyle, NO₂, C(O)NH₂, méthoxy, SCH₃, trifluorométhyle ou méthanesulfonyle ;
R² représente un groupement méthyle, éthyle, propyle, oxygène ou cyano et
R³ représente un groupement trifluorométhyle, méthyle, éthyle, propyle, butyle, isopropyle, tert-butyle, chloro, fluoro, bromo, cyano, méthoxy, acétyle, trifluorométhanesulfonyle, trifluorométhoxy ou trifluorométhylthio.

3. Emploi conforme à l'une quelconque des revendications 1 à 2, où le composé de formule I est également de formule II ci-après, ou de ses sels, formes polymorphiques, solvates, hydrates, métabolites, promédicaments ou formes diastéréoisomères ; 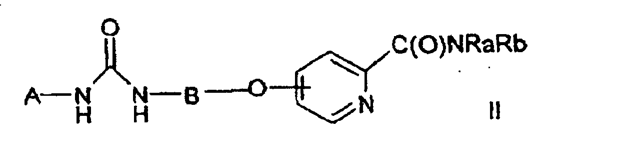 où
Ra et Rb représente indépendamment un atome d'hydrogène ou un groupement alkyle en C₁-C₄,
B dans la formule II représente 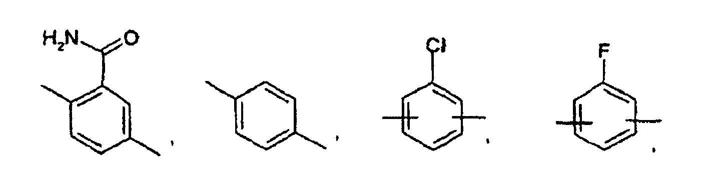 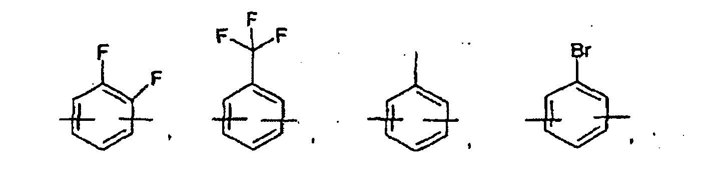 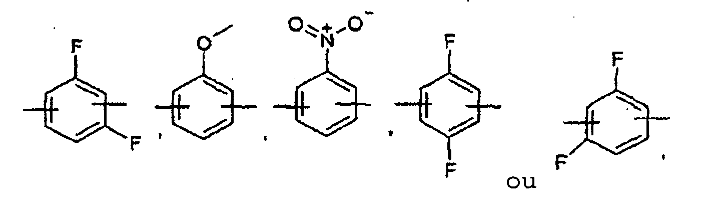 où le groupement urée, -NH-C(O)-NH-, et le groupement pontant oxygène ne sont pas liés à des carbones de cycle contigus de B, mais sont séparés par 1 ou 2 atomes de carbone de cycle, et
A dans la formule (II) représente 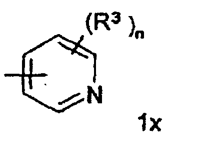 ou 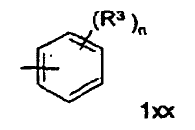 où la variable n est égale à 0, 1, 2, 3 ou 4, et
R³ représente un groupement trifluorométhyle, méthyle, éthyle, propyle, butyle, isopropyle, tert-butyle, chloro, fluoro, bromo, cyano, méthoxy, acétyle, trifluorométhanesulfonyle, trifluorométhoxy ou trifluorométhylthio.

4. Emploi conforme à l'une quelconque des revendications 1 à 3 où chacun des substituants R³ représente un atome de chlore ou un groupement trifluorométhyle, tert-butyle ou méthoxy,
A dans la formule II représente 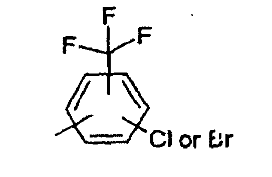 et
B dans la formule II représentent un groupement phénylène, phénylène fluoré ou phénylène difluoré.

5. Emploi conforme à l'une quelconque des revendications 1 à 4, où le composé de formule I est également de formule X ci-après, ou de ses sels, formes polymorphiques, solvates, hydrates, métabolites, promédicaments ou formes diastéréoisomères ; 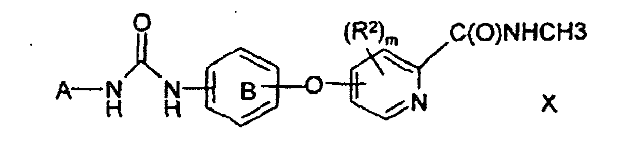 où le cycle phénylique « B » comporte éventuellement un substituant halogéné, A représente un groupement phényle éventuellement substitué de formule 1xx : 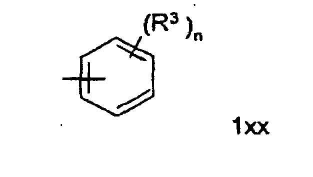 un groupement pyridinyle éventuellement substitué de formule 1x : 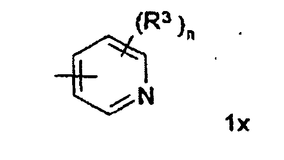 ou un groupement naphtyle éventuellement substitué de formule 1y : 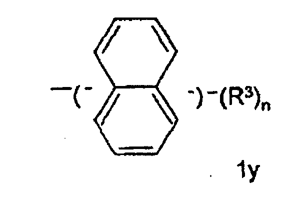 n est égal à 0, 1, 2, 3, 4, 5 ou 6,
m est égal à 0, 1, 2 ou 3,
chacun des groupements R² représente indépendamment : un groupement alkyle en C₁₋₅, halogénoalkyle en C₁₋₅, alkoxy en C₁₋₃, N-oxo ou N-hydroxy,
chacun des groupements R³ représente indépendamment : un atome d'halogène ou un groupement R⁴ , OR⁴ , S (O)R⁴ , C(O)R⁴, C(O)NR⁴R⁵, oxo, cyano ou nitro (NO₂) et
R⁴ et R⁵ représentent indépendamment un atome d'hydrogène ou un groupement alkyle en C₁₋₆, ou un groupement alkyle en C₁₋₆ substitué par des atomes d'halogène, éventuellement perhalogéné.

6. Emploi conforme à la revendication 5 où m est égal à zéro et A représente un groupement phényle éventuellement substitué par au moins un substituant R³.

7. Emploi conforme à la revendication 6 où R³ représente un atome d'halogène ou un groupement trifluorométhyle et/ou méthoxy.

8. Emploi conforme à la revendication 1 où le composé de formule I présente également la structure de l'une des formules Z1 ou Z2 ci-après, ou de l'un de leurs sels, formes polymorphiques, solvates, hydrates, métabolites, promédicaments ou formes diastéréoisomères : 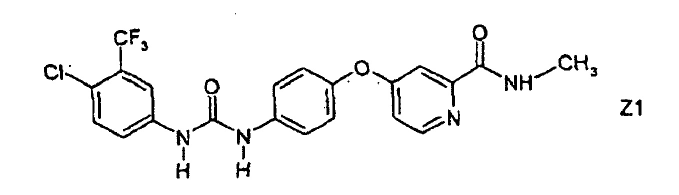 ou

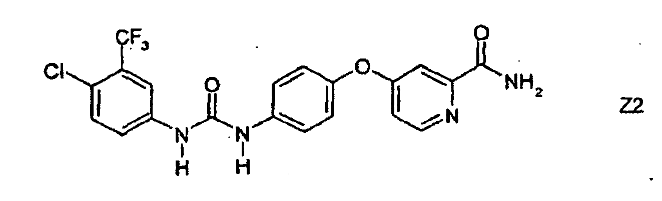

9. Emploi conforme à la revendication 8 où le composé de formule I est le sel de tosylate du composé de formule Z1.

10. Combinaison comprenant au moins un composé de formule I conforme à l'une quelconque des revendications 1 à 9 et au moins un inhibiteur d'élastase et/ou un inhibiteur de kinase.

11. Combinaison conforme à la revendication 10 où l'inhibiteur de kinase est le glivec.

12. Combinaison comprenant au moins un composé de formule I conforme à l'une quelconque des revendications 1 à 9 ou une combinaison conforme à l'une quelconque des revendications 10 à 11 et au moins un agent thérapeutique choisi dans le groupe constitué par les suivants : anticoagulants, diurétique, glycosides cardiotoniques, bloqueurs des canaux calciques, vasodilatateurs, analogues de prostacycline, antagonistes d'endothéline, inhibiteurs de phosphodiestérase, inhibiteurs d'endopeptidase, agents diminuant la lipidémie, inhibiteurs de thromboxane et autres agents thérapeutiques connus pour réduire la pression artérielle pulmonaire.

13. Combinaison conforme à la revendication 12 où l'agent thérapeutique supplémentaire est un inhibiteur de phosphodiestérase V, un antagoniste d'endothéline ou un analogue de prostacycline.

14. Combinaison conforme à la revendication 12 où l'agent thérapeutique supplémentaire est le suivant : tadalafil, sildénafil, vardénafil, bosentane, sitaxentane, ilomédine, tréprostinil et époprosténol.

15. Emploi de la combinaison conforme à l'une quelconque des revendications 10 à 14 dans la fabrication d'un médicament destiné au traitement prophylactique ou thérapeutique ou à la gestion de l'hypertension pulmonaire.

16. Composition pharmaceutique comprenant une combinaison conforme à l'une quelconque des revendications 10 à 14.

17. Composition pharmaceutique conforme à la revendication 16 destinée au traitement de l'hypertension pulmonaire.
